# EUROPÄISCHE PATENTANMELDUNG

(11) **EP 3 816 122 A1**
(43) Veröffentlichungstag der Anmeldung: **05.05.2021**
(21) Anmeldenummer: 20191378.7
(22) Anmeldetag: 17.08.2020
(51) Int. Cl.: C03C 13/04, C03C 25/105, C03C 25/40, G02B 1/10, A61B 1/00

(54) **OPTISCHER FASERARTIKEL, SEINE HERSTELLUNG UND VERWENDUNG**

(30) Priorität: 30.09.2019 DE 102019126259
(71) Anmelder: Schott AG, 55122 Mainz (DE)
(72) Erfinder: Mangold, Stephanie, 55288 Schornsheim (DE); Weingärtner, Thomas, 55435 Gau-Algesheim (DE); Fratzer, Oliver, 63329 Egelsbach (DE)
(74) Vertreter: Fuchs Patentanwälte Partnerschaft mbB

(57) **Zusammenfassung**

Die vorliegende Erfindung betrifft einen optischen Faserartikel und ein Verfahren zu Herstellung des optischen Faserartikels. Die vorliegende Erfindung betrifft insbesondere die Verwendung des optischen Faserartikels in einem Faserbündel als Lichtlichter und/oder Bildleiter, beispielsweise in einem Endoskop.

## Beschreibung

Die vorliegende Erfindung betrifft einen optischen Faserartikel und ein Verfahren zu Herstellung des optischen Faserartikels. Die vorliegende Erfindung betrifft auch die Verwendung des optischen Faserartikels in einem Faserbündel als Lichtlichter und/oder Bildleiter, beispielsweise in einem Endoskop.

Optische Faserartikel wie beispielsweise Glasfaserartikel können als Lichtleiter und/oder Bildleiter Daten übertragen und/oder Licht transportieren. Optische Faserartikel werden häufig im medizinischen Bereich beispielsweise für therapeutische und/oder diagnostische Verfahren verwendet. Lichtleiter und/oder Bilderleiter kommen beispielsweise für den flexiblen Transport von Licht in Messgeräten, Mikroskopen, Spektroskopen, Inspektionskameras und Endoskopen zum Einsatz. Medizinische Geräte wie Endoskope werden sehr häufig autoklaviert und/oder sterilisiert. Bei der Dampfsterilisation werden die Materialien hohen Drücken, Temperaturen und gleichzeitig hoher Luftfeuchtigkeit ausgesetzt. Lichtlichter und/oder Bildleiter für den Einsatz in medizinischen Geräten wie beispielsweise einem Endoskop müssen daher eine gute Autoklavierbarkeit bzw. Sterilisierbarkeit und hohe Korrosionsbeständigkeit aufweisen, um langfristig verwendbar zu bleiben. Daher werden optische Faserartikel benötigt, die die Langlebigkeit von medizinischen Geräten erhöhen und zum verringerten Auftreten von Korrosion führen. Weiterhin wären optische Faserartikel wünschenswert, die eine hohe Temperaturbeständigkeit und Luftfeuchteresistenz aufweisen. Gleichzeitig sollte der optischen Faserartikel eine hohe Biegsamkeit und gute mechanische Stabilität aufweisen insbesondere für den Einsatz in Endoskopen.

Optische Fasern werden oft mit Schlichten als Schutzschicht bedeckt. Üblicherweise werden optische Fasern mit polymerisierbaren Beschichtungszusammensetzungen versehen, die bei UV-Bestrahlung aushärten. Solche Beschichtungszusammensetzungen enthalten häufig Acrylate, Methacrylate, und andere polymerisierbare Komponenten. Solche polymerisierbaren Beschichtungszusammensetzungen sind jedoch gesundheitlich nicht unbedenklich. Darüber hinaus enthalten solche Beschichtungen Fotoinitiatoren, deren Auswirkungen für die menschliche Gesundheit immer kritischer gesehen werden. Die Verwendung von solchen fotopolymerisierbaren Substanzen in medizinischen Geräten, welche insbesondere mit menschlichen Organen in Kontakt kommen, ist kritisch. Wünschenswert wären daher optische Faserartikel, die ohne gesundheitlich bedenkliche Komponenten herstellbar sind. Die Materialien sollten insbesondere möglichst gering bis gar nicht toxisch sein und keine Gefahrenquelle für den Patienten darstellen. Die Bereitstellung von optischen Fasern mit verbesserter Biokompatibilität und verringerter Toxizität wäre daher wünschenswert.

US 2003/0045600 A1 beschreibt eine Schlichte, welche Silane wie beispielsweise Polyalkoxysilane und Polyhalosilane umfassen kann. Die Schlichte der US 2003/0045600 A1 kann auch Halogenide wie Chlor oder Fluor enthalten. Weiterhin beschreibt die US 2003/0045600 A1 eine Schlichte, die als Hauptbestandteil Polyether-Urethan-Acrylat enthalten kann.

WO 01/49625 A1 beschreibt eine optische Faser mit einer UV-härtbaren Schlichte, die fotopolymerisierbare Verbindungen wie beispielsweise Caprolacton-(meth)acrylat oder 4-Hydroxybutyl(meth)acrylat enthalten kann. Weiterhin kann die Schlichte der WO 01/49625 A1 Fotoinitiatoren wie beispielsweise 1-Hydroxycyclohexylphenylketon oder 2,2-Dimethoxy-2-phenylacetophenon enthalten.

Es ist daher eine Aufgabe der vorliegenden Erfindung, die Nachteile aus dem Stand der Technik zu überwinden. Insbesondere ist es eine Aufgabe, einen verbesserten optischen Faserartikel für die Verwendung in medizinischen und diagnostischen Geräten wie beispielsweise Endoskopen bereitzustellen. Der optische Faserartikel soll sich insbesondere durch eine hohe Korrosionsbeständigkeit auch in der Dampfsterilisation, eine lange Nutzungsdauer, eine gute Biegebelastbarkeit, sowie eine besonders hohe Biokompatibilität und verringerte Toxizität auszeichnen.

Die Aufgaben werden durch die Gegenstände der Patentansprüche gelöst. Die Aufgaben werden insbesondere gelöst durch einen optischen Faserartikel, umfassend mindestens eine optische Faser und eine auf deren Oberfläche angeordnete Funktionsschicht. Die optische Faser kann einen Faserkern und einen auf dem Faserkern angeordneten Mantel aufweisen.

Die Funktionsschicht kann mindestens ein funktionelles Silan mit folgender Strukturformel umfassen: 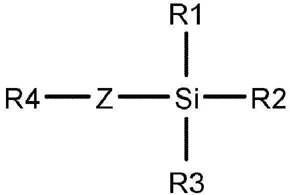
wobei Z eine verzweigte oder unverzweigte Alkylgruppe oder Arylgruppe mit 1 bis 18 Kohlenstoffatomen ist,
wobei R1, R2 und R3 unabhängig voneinander ausgewählt sind aus Wasserstoff, Sauerstoff, Alkyl, Hydroxyalkyl und Hydroxyl, und wobei eine, zwei oder drei der Gruppen R1, R2 oder R3 über eine kovalente Bindung direkt oder indirekt mit der Oberfläche verbunden ist,
und wobei R4 ausgewählt ist aus, -NH₂, -NHR', -NR'R", Glycidyloxy und -SH, wobei R' und R" unabhängig voneinander ausgewählt sind aus Alkyl, Aminoalkyl, Hydroxyalkyl und -(CH₂)ₘNH₂ mit m von 1 bis 6.

In einem Aspekt betrifft die Erfindung einen optischen Faserartikel umfassend mindestens eine optische Faser und eine auf der Oberfläche der optischen Faser angeordnete Funktionsschicht, wobei die Funktionsschicht im IR-Spektrum durch folgende Absorption gekennzeichnet ist:
a. ein Verhältnis der maximalen Absorptionsbandenhöhe im Bereich von 800 cm⁻¹ bis 1200 cm⁻¹ zu der maximalen Absorptionsbandenhöhe im Bereich von 1500 cm⁻¹ bis 1900 cm⁻¹ ist mindestens 2,0;
b. ein Verhältnis der maximalen Absorptionsbandenhöhe im Bereich von 2700 cm⁻¹ bis 3000 cm⁻¹ zu der maximalen Absorptionsbandenhöhe im Bereich von 1500 cm⁻¹ bis 1900 cm⁻¹ ist mindestens 2,0; und
c. ein Verhältnis der maximalen Absorptionsbandenhöhe im Bereich von 800 cm⁻¹ bis 1200 cm⁻¹ zu der maximalen Absorptionsbandenhöhe im Bereich von 2700 cm⁻¹ bis 3000 cm⁻¹ ist mindestens 1,1 und höchstens 2,0.

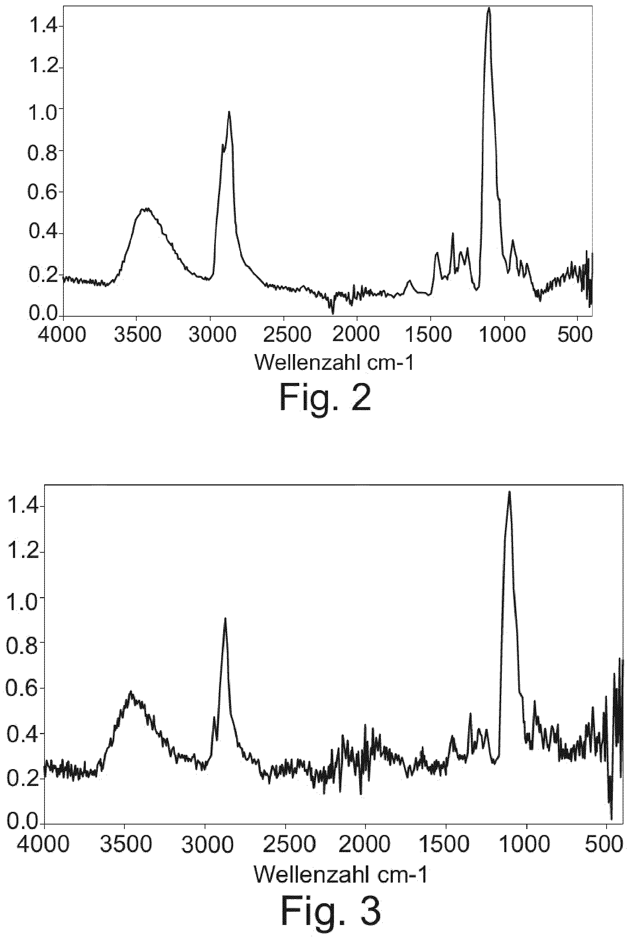

Der optische Faserartikel mit einem solchen IR-Spektrum wird erhalten, wenn hierin beschriebene Maßnahmen umgesetzt werden. In besonders bevorzugten Ausführungen hat die Funktionsschicht ein IR-Spektrum im Wesentlichen gemäß Figur 2 oder Figur 3.

"Optische Faser" meint eine Faser, welche Licht über kurze oder lange Strecken transportieren kann. Eine solche optische Faser kann beispielsweise eine Glasfaser sein. Die optische Faser umfasst eine Kernschicht (auch "Faserkern"), eine Mantelschicht (auch kurz "Mantel") und eine an der Oberfläche der Mantelschicht angeordnete Funktionsschicht.

"Optisches Faserbündel" meint eine Mehrzahl von optischen Fasern. Beispielsweise kann ein optisches Faserbündel aus 10 oder mehr optischen Fasern bestehen.

"Optischer Faserartikel" meint einen Artikel, der mindestens eine optische Faser umfasst. Der optische Faserartikel kann auch eine Vielzahl von optischen Fasern umfassen. Optische Fasern und optische Faserartikel können beispielsweise in Lichtleitern und/oder Bildleitern verwendet werden. Für die Verwendung in solchen Lichtleitern und/oder Bildleitern können beispielsweise mehrere optische Faserartikel miteinander verklebt und zusammen in einer Hülse eingebettet werden.

"Funktionsschicht" meint eine auf der Oberfläche der Mantelschicht angeordnete Schicht, welche die optische Faser mindestens teilweise, insbesondere im Wesentlichen vollständig, bedeckt. Die Funktionsschicht dient unter anderem dem Schutz der optischen Faser beispielsweise vor Brüchen. Die Funktionsschicht kann beispielsweise aus einer Schlichte gebildet sein.

"Schlichte" meint eine Zusammensetzung, die dazu dient, die Funktionsschicht auf optische Fasern aufzubringen. Sie kann nach der Herstellung der Faser z.B. durch Eintauchen, Besprühen oder in einem Roll-to-roll-Verfahren aufgetragen werden.

"Funktionelles Silan" meint eine Silanverbindung, welche mindestens eine funktionelle Gruppe umfasst, welche chemische Reaktionen eingehen kann. Eine solche funktionelle Gruppe kann beispielsweise eine Aminogruppe sein. Das funktionelle Silan kann beispielsweise als Haftvermittler dienen und als Brücke zwischen der Oberfläche der Mantelschicht und einem Klebstoff fungieren.

"Mantelschicht" meint eine Schicht, welche unterhalb der Funktionsschicht angeordnet ist und welche eine Kernschicht umgibt. Die Mantelschicht kann beispielsweise Glas oder ein Polymer wie beispielsweise Polyimid enthalten. Die Mantelschicht trägt insbesondere zur Lichttransportfunktion der optischen Faser bei.

"Kernschicht" meint die innere Schicht der optischen Faser und ist von der Mantelschicht umgeben. Die Kernschicht kann beispielsweise Glas oder ein Polymer wie beispielsweise Polycarbonat enthalten. Die Kernschicht trägt zusammen mit der Mantelschicht insbesondere zur Lichttransportfunktion der optischen Faser bei.

Die folgende detaillierte Beschreibung nennt Maßnahmen und Merkmale, die zum erfindungsgemäßen Erfolg beitragen können. Dabei ist es nicht erforderlich, alle Maßnahmen in einem optischen Faserartikel umzusetzen. Vielmehr lassen sich beabsichtigte Verbesserungen bereits erreichen, wenn nur einige der nachfolgenden Maßnahmen angewendet werden.

In einer Ausführungsform weist die Funktionsschicht ein funktionelles Silan auf. Das funktionelle Silan weist freie funktionelle Gruppen auf, wie beispielsweise Aminogruppen, die nicht durch Reaktion mit Acrylaten oder anderen reaktiven Verbindungen abreagiert sind. Das funktionelle Silan vermittelt eine gute Haftung der Funktionsschicht an die Oberfläche der Mantelschicht. Eine gute Haftung der Funktionsschicht an der optischen Faser dient der Schutzwirkung der Funktionsschicht wie beispielsweise dem Schutz vor Mikrorissen und Brüchen insbesondere bei wiederholter und erhöhter Biegebelastung des Faserartikels. Zusätzlich verleiht das funktionelle Silan der Funktionsschicht eine gute Haftvermittlerfunktion an der von der optischen Faser abgewandten Seite. Die Funktionsschicht ist hierdurch in der Lage, eine gute Haftung mit einem weiteren Material wie beispielsweise einem Kleber einzugehen. Durch das funktionelle Silan wird eine gute Verklebbarkeit des optischen Faserartikels erreicht. Werden beispielsweise mehrere optische Faserartikel in einer Hülse eingebettet und verklebt, wird eine bessere Haftung der optischen Faserartikel sowohl untereinander als auch an der Hülse erreicht. Eine solche Hülse kann beispielsweise Edelstahl oder Kunststoff enthalten.

Bevorzugt weist die Alkylgruppe in Z mindestens ein Kohlenstoffatom auf, mindestens 2 Kohlenstoffatome, mindestens 3 Kohlenstoffatome, oder mindestens 4 Kohlenstoffatome. Bevorzugt weist die Alkylgruppe in Z höchstens 25 Kohlenstoffatom auf, weiter bevorzugt höchstens 20 Kohlenstoffatome, weiter bevorzugt höchstens 15 Kohlenstoffatome, weiter bevorzugt mindestens höchstens 12 Kohlenstoffatome, weiter bevorzugt höchstens 10 Kohlenstoffatome, weiter bevorzugt höchstens 9 Kohlenstoffatome, weiter bevorzugt höchstens 8 Kohlenstoffatome, weiter bevorzugt höchstens 7 Kohlenstoffatome. In Ausführungsformen weist die Alkylgruppe in Z 1 bis 25 Kohlenstoffatome auf, weiter bevorzugt 1 bis 20 Kohlenstoffatome, weiter bevorzugt 2 bis 15 Kohlenstoffatome oder 3 bis 12 Kohlenstoffatome. In besonders bevorzugten Ausführungsformen weist die Alkylgruppe in Z 3 bis 8 Kohlenstoffatome auf. Die Anzahl an Kohlenstoffatomen beeinflusst die Gleiteigenschaften der optischen Fasern. Hierdurch können die optischen Fasern bzw. der optische Faserartikel häufiger und stärker gebogen werden ohne zu brechen. Verbesserte Gleiteigenschaften führen ebenfalls dazu, dass die optischen Fasern gut aneinander gleiten und gleichzeitig nicht miteinander verkleben. Bevorzugt ist die Alkylgruppe in Z eine unverzweigte Alkylgruppe.

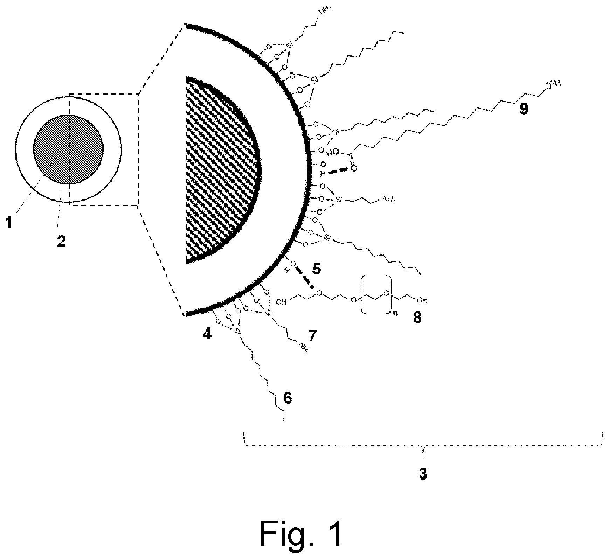

Die Gruppen R1, R2 und R3 können unabhängig voneinander ausgewählt sein aus Wasserstoff, Sauerstoff, Alkyl, Alkyloxy, Hydroxyalkyl und Hydroxyl, wobei eine, zwei oder drei der Gruppen R1, R2 oder R3 über eine kovalente Bindung direkt oder indirekt mit der Oberfläche der optischen Faser verbunden sind. Mit anderen Worten kann beispielsweise eine der Gruppen R1, R2 oder R3 eine Alkylgruppe (z.B. Methylgruppe) oder eine Alkyloxygruppe (z.B. Methoxygruppe) sein und zwei weitere Gruppen sind Sauerstoff und über eine Siloxangruppe an die Oberfläche der Faser gebunden (vgl. Figur 1). Der Alkylbestandteil von R1, R2 und/oder R3 kann eine Kettenlänge von 1 bis 6, insbesondere 1 bis 3 Kohlenstoffatomen aufweisen. Insbesondere sind eine, zwei oder drei der Gruppen R1, R2 oder R3 des funktionellen Silans über eine kovalente Bindung direkt oder indirekt mit der Oberfläche der Faser verbunden. Bevorzugt ist eine, zwei oder drei der Gruppen R1, R2 und R3 eine (-X-R''') Gruppe, wobei X ausgewählt ist aus Sauerstoff, Selen und Schwefel und wobei R'" ausgewählt ist aus einer Alkylgruppe, Wasserstoff und der Oberfläche der Faser. Weiter bevorzugt ist eine, zwei oder drei der Gruppen R1, R2 und R3 eine (-X-R''') Gruppe, wobei X Sauerstoff ist und wobei R'" ausgewählt ist aus einer Alkylgruppe, Wasserstoff und der Oberfläche der Faser. Weiter bevorzugt ist eine, zwei oder drei der Gruppen R1, R2 und R3 eine (-X-R''') Gruppe, wobei X Sauerstoff ist und wobei R'" die Oberfläche der Faser ist. Weiter bevorzugt sind zwei oder drei der Gruppen R1, R2 und R3 eine (-X-R''') Gruppe, wobei X Sauerstoff ist und wobei R'" die Oberfläche der Faser ist. Die Alkylgruppe kann eine Kohlenstoffkettenlänge im Bereich von 1 bis 6, insbesondere von 1 bis 3 Kohlenstoffatomen haben. Beispielsweise bildet das funktionelle Silan eine oder zwei oder drei Siloxanverbindungen mit der Oberfläche der Mantelschicht aus. Das hier beschriebene funktionelle Silan trägt hierdurch wesentlich zur Haftung der Funktionsschicht an der optischen Faser bei.

R4 kann ausgewählt sein aus, -NH₂, -NHR', -NR'R", Glycidyloxy und -SH, wobei R' und R" unabhängig voneinander ausgewählt sind aus Alkyl, Aminoalkyl, Hydroxyalkyl und -(CH₂)ₘNH₂ mit m von 1 bis 6, insbesondere 1 bis 3. In bevorzugten Ausführungsformen ist R4 ausgewählt aus, -NH₂, -NHR', -NR'R", wobei R' und R" unabhängig voneinander ausgewählt sind aus Alkyl, Aminoalkyl, Hydroxyalkyl und -(CH₂)ₘNH₂ mit m von 1 bis 6, weiter bevorzugt ist m von 1 bis 5, weiter bevorzugt von 1 bis 4, weiter bevorzugt von 1 bis 3, weiter bevorzugt von 1 bis 2. Besonders bevorzugt ist R4 ausgewählt aus, -NH₂ und -NHR', wobei R' ausgewählt ist aus Alkyl, Aminoalkyl, Hydroxyalkyl und -(CH₂)ₘNH₂ mit m von 1 bis 6, weiter bevorzugt ist m von 1 bis 5, weiter bevorzugt von 1 bis 4, weiter bevorzugt von 1 bis 3, weiter bevorzugt von 1 bis 2. Besonders bevorzugt ist R4 ausgewählt aus -NH₂ und -NHR', wobei R' Alkyl ist. In einer besonders bevorzugten Ausführungsform ist R4 -NH₂. In einer besonders bevorzugten Ausführungsform ist R4 -(CH₂)₂NH₂. Das funktionelle Silan mit einer solchen Gruppe R4 ermöglicht eine gute Haftvermittlung und Verklebung, beispielsweise wenn optische Faserartikel in eine Hülse mit einem Klebstoff eingebettet werden.

In einer besonders bevorzugten Ausführungsformen weist das funktionelle Silan folgende Strukturformel auf: 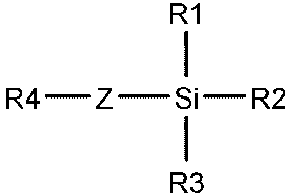 wobei R1, R2 und R3 eine (-X-R''') Gruppe ist, wobei X Sauerstoff ist und wobei R''' die Oberfläche der Faser ist; wobei Z eine unverzweigte Alkylgruppe mit 3 Kohlenstoffatomen ist; und wobei R4 ausgewählt aus, -NH₂ und -NHR' wobei R"-(CH₂)ₘNH₂ mit m = 2 ist.

In einer besonders bevorzugten Ausführungsformen weist das funktionelle Silan folgende Strukturformel auf: 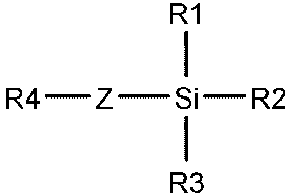 wobei R1, R2 und R3 eine (-X-R''') Gruppe ist; wobei X Sauerstoff ist und wobei R''' die Oberfläche der Faser ist; wobei R4 -NH₂ ist; und wobei Z eine unverzweigte Alkylgruppe mit 1 bis 10 Kohlenstoffatomen ist, weiter bevorzugt, mit 2 bis 9 Kohlenstoffatomen, weiter bevorzugt mit 3 bis 8 Kohlenstoffatomen.

In einer anderen besonders bevorzugten Ausführungsformen weist das funktionelle Silan folgende Strukturformel auf: 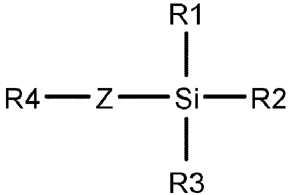 wobei R1, R2 und R3 eine (-X-R''') Gruppe ist, wobei X Sauerstoff ist und wobei R''' die Oberfläche der Faser ist; wobei R1, R2 und R3 über eine kovalente Bindung direkt mit der Oberfläche der Faser verbunden ist; wobei R4 -NHR' ist; wobei R' -(CH₂)ₘNH₂ mit m = 2 ist; und wobei Z eine unverzweigte Alkylgruppe mit 1 bis 10 Kohlenstoffatomen ist, weiter bevorzugt, von 2 bis 9 Kohlenstoffatomen, weiter bevorzugt von 3 bis 8 Kohlenstoffatomen.

In einer bevorzugten Ausführungsform ist Z=3 und R4 ist NH₂ oder NHR' mit R' = -(CH₂)ₘNH₂ und m = 2. Beispiele für erfindungsgemäß bevorzugt eingesetzte funktionelle Silane sind N-[3-(Trimethoxysilyl)propyl]ethylenediamin, 3-Aminopropyl-diethoxy-methylsilan, 3-Glycidyloxypropyl-triethoxysilan, 3-Glycidoxypropyldimethoxymethylsilane und Kombinationen davon bzw. deren Reaktionsprodukte mit der Oberfläche der Faser, insbesondere deren mit der Oberfläche der Faser über eine, zwei oder drei kovalente Bindungen verbundene Derivate.

In bestimmten Ausführungsformen umfasst die Funktionsschicht eine Mehrzahl von funktionellen Silanen. In bestimmten Ausführungsformen umfasst die Funktionsschicht mindestens zwei funktionelle Silane, mindestens drei funktionelle Silane, oder mindestens vier funktionelle Silane.

Bevorzugt umfasst die Funktionsschicht das funktionelle Silan mit einem Anteil von mindestens 0,1 Gew.-%, weiter bevorzugt von mindestens 0,5 Gew.-%, weiter bevorzugt von mindestens 0,9 Gew.-%, weiter bevorzugt von mindestens 1,2 Gew.-%, weiter bevorzugt von mindestens 1,5 Gew.-%, weiter bevorzugt von mindestens 1,8 Gew.-%, weiter bevorzugt von mindestens 2 Gew.-%. Bevorzugt umfasst die Funktionsschicht das funktionelle Silan mit einem Anteil von höchstens 70 Gew.-%, weiter bevorzugt von höchstens 60 Gew.-%, weiter bevorzugt von höchstens 50 Gew.-%, weiter bevorzugt von höchstens 40 Gew.-%, weiter bevorzugt von höchstens 30 Gew.-%, weiter bevorzugt von höchstens 25 Gew.-%, weiter bevorzugt von höchstens 15 Gew.-%. In bevorzugten Ausführungsformen umfasst die Funktionsschicht das funktionelle Silan mit einem Anteil von 0,1 bis 70 Gew.-%, weiter bevorzugt von 0,5 bis 50 Gew.-%, weiter bevorzugt von 0,9 bis 40 Gew.-%, weiter bevorzugt von 1,2 bis 30 Gew.-%, weiter bevorzugt von 1,5 bis 15 Gew.-%. In besonders bevorzugten Ausführungsformen umfasst die Funktionsschicht das funktionelle Silan mit einem Anteil von 1 bis 40 Gew.-%, oder von 2 bis 15 Gew.-%. Ist der Anteil an funktionellem Silan zu niedrig, haftet die Funktionsschicht schlechter an der Oberfläche der Mantelschicht, worauf die optische Faser leichter Mikrorisse bildet und leichter bricht. Ist der Anteil an funktionellem Silan zu hoch, gleiten die optischen Fasern schlechter aneinander und neigen dazu, aneinander zu haften. Dies führt zu einer erhöhten Bildung von Mikrorissen und Brüchen bei wiederholten und erhöhten Biegebelastungen. Es ist anzumerken, dass der Gehalt an funktionellem Silan in der Funktionsschicht im Vergleich zu Funktionsschichten des Standes der Technik vergleichsweise hoch ist, da die zur Herstellung der Funktionsschicht eingesetzten Schlichten vorzugsweise im Wesentlichen keine polymerisierbaren Lösungsmittel, wie Acrylate oder Methacrylate, enthalten. Die in den Schlichten eingesetzten Lösungsmittel verbleiben nach Trocknung nicht in der Funktionsschicht.

In Ausführungsformen umfasst die Funktionsschicht alternativ oder zusätzlich zu dem funktionellen Silan mindestens ein Alkylsilan. Bevorzugt umfasst die Funktionsschicht ein kovalent an die Oberfläche der Faser gebundenes Alkylsilan. Bevorzugt weist das Alkylsilan folgende Strukturformel auf: 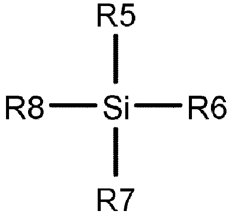
wobei R5, R6 und R7 unabhängig voneinander ausgewählt sind aus Wasserstoff, Sauerstoff, Alkyl, Alkyloxy, Hydroxyalkyl und Hydroxyl, und wobei eine, zwei oder drei der Gruppen R5, R6 oder R7 über eine kovalente Bindung direkt oder indirekt mit der Oberfläche der Faser verbunden ist,
wobei R8 ausgewählt ist aus verzweigter und unverzweigter Alkylgruppe mit 1 bis 25 Kohlenstoffatomen. Das Alkylsilan verbessert die hydrolytische Beständigkeit der optischen Faser und verringert die Oberflächenenergie.

Bevorzugt weist die Alkylgruppe in R8 des Alkylsilans mindestens ein Kohlenstoffatom auf, mindestens 2 Kohlenstoffatome, mindestens 5 oder mindestens 8 Kohlenstoffatome. Bevorzugt weist die Alkylgruppe in R8 höchstens 25 Kohlenstoffatom auf, weiter bevorzugt höchstens 20 Kohlenstoffatome, weiter bevorzugt höchstens 15 Kohlenstoffatome, weiter bevorzugt mindestens höchstens 12 Kohlenstoffatome, weiter bevorzugt höchstens 10 Kohlenstoffatome. In Ausführungsformen weist die Alkylgruppe in R8 1 bis 25 Kohlenstoffatome auf, weiter bevorzugt 1 bis 20 Kohlenstoffatome, weiter bevorzugt 1 bis 15 Kohlenstoffatome, weiter bevorzugt 3 bis 12 Kohlenstoffatome. In besonders bevorzugten Ausführungsformen weist die Alkylgruppe in R8 5 bis 10 Kohlenstoffatome, oder 8 bis 15 Kohlenstoffatome auf. Die erfindungsgemäße Kettenlänge des Alkylsilans verbessert das Aneinandergleiten der optischen Fasern beispielsweise innerhalb eines optischen Faserbündels und verhindert das Verkleben der optischen Fasern untereinander. Dies führt zu einer verbesserten Handhabbarkeit und Wiedervereinzelbarkeit der optischen Fasern in optischen Faserbündeln. Bevorzugt ist die Alkylgruppe in R8 des Alkylsilans eine unverzweigte Alkylgruppe. Bevorzugt ist eine, zwei oder drei der Gruppen R5, R6 und R7 eine (-X-R''') Gruppe, wobei X ausgewählt ist aus Sauerstoff, Selen und Schwefel und wobei R''' ausgewählt ist aus einer Alkylgruppe, Wasserstoff und der Oberfläche der Faser. Weiter bevorzugt ist eine, zwei oder drei der Gruppen R5, R6 und R7 eine (-X-R''') Gruppe, wobei X Sauerstoff ist und wobei R'" ausgewählt ist aus einer Alkylgruppe, Wasserstoff und der Oberfläche der Faser. Weiter bevorzugt ist eine, zwei oder drei der Gruppen R5, R6 und R7 eine (-X-R''') Gruppe, wobei X Sauerstoff ist und wobei R'" die Oberfläche der Faser ist. Weiter bevorzugt sind zwei oder drei der Gruppen R5, R6 und R7 eine (-X-R''') Gruppe, wobei X Sauerstoff ist und wobei R'" die Oberfläche der Faser ist. Beispielsweise bildet das Alkylsilan eine oder zwei oder drei Siloxanverbindungen mit einer Si-OH-Gruppe der Oberfläche der Faser. Das hier beschriebene Alkylsilan verbessert die Haftung der Funktionsschicht an der Oberfläche der Faser. Weiterhin verbessert das hier beschriebene Alkylsilan die Geschmeidigkeit der optischen Fasern beispielsweise in einem optischen Faserbündel und setzt die gegenseitige Reibung der optischen Fasern herab.

Bevorzugt weist das Alkylsilan folgende Strukturformel auf: 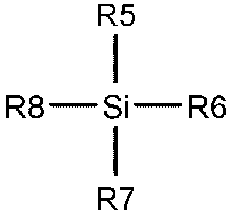 wobei R5, R6 und R7 eine (-X-R''') Gruppe ist; wobei X Sauerstoff ist und wobei R'" die Oberfläche der Faser ist; wobei R5, R6 und R7 über eine kovalente Bindung direkt mit der Oberfläche der Faser verbunden ist; und wobei R8 eine unverzweigte Alkylgruppe mit 1 bis 15 Kohlenstoffatomen ist, weiter bevorzugt mit 1 bis 12 Kohlenstoffatomen, weiter bevorzugt von 1 bis 10 Kohlenstoffatomen, weiter bevorzugt mit 1 bis 9 Kohlenstoffatomen, weiter bevorzugt mit 3 bis 8 oder genau 8 Kohlenstoffatomen.

Beispiele für erfindungsgemäß eingesetzte Alkylsilane sind Trimethoxypropylsilan, Trimethoxyoctylsilan, Trimethoxymethylsilan, n-Propyltriethoxysilan, Triethoxymethylsilan, Dimethoxydimethylsilan, Diethoxydimethylsilan, bzw. deren Reaktionsprodukte mit der Oberfläche der Faser, insbesondere deren mit der Oberfläche der Faser über eine, zwei oder drei kovalente Bindungen verbundene Derivate.

In bestimmten Ausführungsformen umfasst die Funktionsschicht eine Mehrzahl von Alkylsilanen. In bestimmten Ausführungsformen umfasst die Funktionsschicht mindestens zwei Alkylsilane, mindestens drei Alkylsilane, oder mindestens vier Alkylsilane

Bevorzugt umfasst die Funktionsschicht das Alkylsilan mit einem Anteil von mindestens 0,8 Gew.-%, weiter bevorzugt von mindestens 1 Gew.-%, weiter bevorzugt von mindestens 2 Gew.-%, weiter bevorzugt von mindestens 3 Gew.-%, weiter bevorzugt von mindestens 4 Gew.-%, weiter bevorzugt von mindestens 5 Gew.-%, weiter bevorzugt von mindestens 6 Gew.-%. Bevorzugt umfasst die Funktionsschicht mindestens ein Alkylsilan mit einem Anteil von höchstens 65 Gew.-%, weiter bevorzugt von höchstens 60 Gew.-%, weiter bevorzugt von höchstens 55 Gew.-%, weiter bevorzugt von höchstens 50 Gew.-%, weiter bevorzugt von höchstens 45 Gew.-%, weiter bevorzugt von höchstens 40 Gew.-%, weiter bevorzugt von höchstens 35 Gew.-%. In bevorzugten Ausführungsformen umfasst die Funktionsschicht das Alkylsilan mit einem Anteil von 0,8 bis 65 Gew.-%, weiter bevorzugt von 1 bis 60 Gew.-%, weiter bevorzugt von 29 bis 55 Gew.-%, weiter bevorzugt von 3 bis 50 Gew.-%, weiter bevorzugt von 4 bis 45 Gew.-%. In besonders bevorzugten Ausführungsformen umfasst die Funktionsschicht das Alkylsilan mit einem Anteil von 6 bis 35 Gew.-%. Ist der Anteil an Alkylsilan zu niedrig, weisen die optischen Fasern eine verminderte Geschmeidigkeit und verminderte chemische Beständigkeit auf und es kommt zu einer erhöhten Reibung zwischen den optischen Fasern beispielsweise in einem optischen Faserbündel. Hierdurch bilden sich schneller Mikrorisse und Brüche. Weiterhin vermindert ein zu geringer Anteil an Alkylsilan die chemische Stabilität der optischen Faser beispielsweise gegen Säuren. Ist der Anteil an Alkylsilan zu hoch, wird der Zusammenhalt der optischen Fasern untereinander beispielsweise in einem optischen Faserbündel vermindert.

In Ausführungsformen umfasst die Funktionsschicht alternativ oder zusätzlich zu dem funktionellen Silan und/oder dem Alkylsilan ein Polyethylenglykol (PEG)-Silan. Bevorzugt umfasst die Funktionsschicht ein kovalent an die Oberfläche der Faser gebundenes PEG-Silan. Bevorzugt weist das PEG-Silan folgende Strukturformel auf: 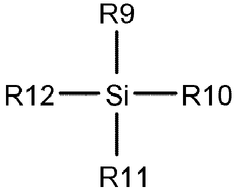
wobei R9, R10 und R11 unabhängig voneinander ausgewählt sind aus Wasserstoff, Sauerstoff, Alkyl, Alkyloxy, Hydroxyalkyl und Hydroxyl, und wobei eine, zwei oder drei der Gruppen R9, R10 und R11 über eine kovalente Bindung direkt oder indirekt mit der Oberfläche der Faser verbunden ist, und
wobei R12 eine Polyethylenglykolgruppe aufweist oder daraus besteht oder ein Derivat davon ist mit einer Kettenlänge von 5 bis 900 Ethylenoxideinheiten. Das PEG-Silan verbessert die hydrolytische Stabilität der optischen Faser.

Bevorzugt weist die Polyethylenglykolgruppe oder Derivat davon in R12 eine Kettenlänge von mindestens 3, weiter bevorzugt von mindestens 4, weiter bevorzugt mindestens von 5, weiter bevorzugt von mindestens 6, weiter bevorzugt von mindestens 7, weiter bevorzugt von mindestens 8 Ethylenoxideinheiten auf. Bevorzugt weist die Polyethylenglykolgruppe oder Derivat davon eine Kettenlänge von höchstens 30, weiter bevorzugt von höchstens 25, weiter bevorzugt von höchstens 20, weiter bevorzugt von höchstens 15, weiter bevorzugt von höchstens 12 Ethylenoxideinheiten auf. In Ausführungsformen weist die Polyethylenglykolgruppe eine Kettenlänge von 3 bis 30, weiter bevorzugt von 4 bis 25, weiter bevorzugt von 5 bis 20 Ethylenoxideinheiten auf. In besonders bevorzugten Ausführungsformen weist die Polyethylenglykolgruppe eine Kettenlänge von 8 bis 12 Ethylenoxideinheiten auf. Die erfindungsgemäße Kettenlänge der Polyethylenglykolgruppe oder des Derivats davon verbessert den Zusammenhalt der optischen Fasern beispielsweise in einem optischen Faserbündel, ohne dass diese miteinander verkleben. Hierdurch verbessert sich die Geschmeidigkeit der optischen Fasern beispielsweise in einem optischen Faserbündel. Die Polyethylenglykolgruppe in R12 kann eine Endgruppe an ihrem freien Ende aufweisen. Die Endgruppe kann eine Hydroxyl- oder Alkyloxygruppe sein, insbesondere eine Methoxygruppe.

Bevorzugt ist eine, zwei oder drei der Gruppen R9, R10 und R11 eine (-X-R''') Gruppe, wobei X ausgewählt ist aus Sauerstoff, Selen und Schwefel und wobei R'" ausgewählt ist aus einer Alkylgruppe, Wasserstoff und der Oberfläche der Faser. Weiter bevorzugt ist eine, zwei oder drei der Gruppen R9, R10 und R11 eine (-X-R''') Gruppe, wobei X Sauerstoff ist und wobei R'" ausgewählt ist aus einer Alkylgruppe, Wasserstoff und der Oberfläche der Faser. Weiter bevorzugt ist eine, zwei oder drei der Gruppen R9, R10 und R11 eine (-X-R''') Gruppe, wobei X Sauerstoff ist und wobei R'" die Oberfläche der Faser ist. Weiter bevorzugt sind zwei oder drei der Gruppen R9, R10 und R11 eine (-X-R''') Gruppe, wobei X Sauerstoff ist und wobei R'" die Oberfläche der Faser ist. Beispielsweise bildet das PEG-Silan eine oder zwei oder drei Siloxanverbindungen mit einer Siliziumgruppe der Oberfläche der Faser. Das hier beschriebene PEG-Silan verbessert die Haftung der Funktionsschicht an der Oberfläche der Faser. Weiterhin verbessert das hier beschriebene PEG-Silan die Geschmeidigkeit der optischen Fasern beispielsweise in einem optischen Faserbündel und setzt die gegenseitige Reibung der optischen Fasern herab.

In besonders bevorzugten Ausführungsformen weist das PEG-Silan folgende Strukturformel auf: 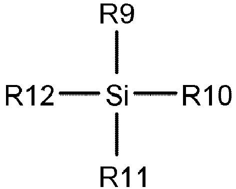 wobei R9, R10 und R11 eine (-X-R''') Gruppe ist; wobei X Sauerstoff ist und wobei R'" die Oberfläche der Faser ist; wobei R9, R10 und R11 über eine kovalente Bindung direkt mit der Oberfläche der Faser verbunden ist; und wobei R12 eine Polyethylenglykolgruppe aufweist, daraus besteht oder ein Derivat davon ist mit einer Kettenlänge von 3 bis 30, weiter bevorzugt von 4 bis 25, weiter bevorzugt von 5 bis 20, weiter bevorzugt von 8 bis 12 Ethylenoxideinheiten.

Beispiele für erfindungsgemäß eingesetzte PEG-Silane sind 2-[Methoxy(polyethyleneoxy)₉₋₁₂propyl]trimethoxysilan, [Hydroxy(polyethyleneoxy)propyl]triethoxysilan, sowie deren Reaktionsprodukte mit der Oberfläche der Faser, insbesondere deren mit der Oberfläche der Faser über eine, zwei oder drei kovalenten Bindungen verbundenen Derivate.

In bestimmten Ausführungsformen umfasst die Funktionsschicht eine Mehrzahl von PEG-Silanen. In bestimmten Ausführungsformen umfasst die Funktionsschicht mindestens zwei PEG-Silane, mindestens drei PEG-Silane, oder mindestens vier PEG-Silane.

Bevorzugt umfasst die Funktionsschicht mindestens ein PEG-Silan mit einem Anteil von mindestens 1 Gew.-%, weiter bevorzugt von mindestens 5 Gew.-%, weiter bevorzugt von mindestens 8 Gew.-%, weiter bevorzugt von mindestens 11 Gew.-%, weiter bevorzugt von mindestens 13 Gew.-%, weiter bevorzugt von mindestens 15 Gew.-%, weiter bevorzugt von mindestens 21 Gew.-%. Bevorzugt umfasst die Funktionsschicht mindestens ein PEG-Silan mit einem Anteil von höchstens 90 Gew.-%, weiter bevorzugt von höchstens 85 Gew.-%, weiter bevorzugt von höchstens 80 Gew.-%, weiter bevorzugt von höchstens 75 Gew.-%, weiter bevorzugt von höchstens 70 Gew.-%, weiter bevorzugt von höchstens 65 Gew.-%, weiter bevorzugt von höchstens 62 Gew.-%. In bevorzugten Ausführungsformen umfasst die Funktionsschicht das PEG-Silan mit einem Anteil von 1 bis 90 Gew.-%, weiter bevorzugt von 5 bis 85 Gew.-%, weiter bevorzugt von 8 bis 80 Gew.-%, weiter bevorzugt von 11 bis 75 Gew.-%, weiter bevorzugt von 13 bis 70 Gew.-%. In einer besonders bevorzugten Ausführungsform umfasst die Funktionsschicht das PEG-Silan mit einem Anteil von 21 bis 65 Gew.-%. Ist der Anteil an PEG-Silan zu niedrig, kommt es zu einem verminderten Zusammenhalt der optischen Fasern beispielsweise in einem optischen Faserbündel. Ist der Anteil an PEG-Silan zu hoch, wird der Zusammenhalt der optischen Fasern untereinander beispielsweise in einem optischen Faserbündel vermindert. Weiterhin vermindert ein zu geringer Anteil an PEG-Silan die chemische Stabilität der optischen Faser. Ist der Anteil an PEG-Silan zu hoch, neigen die optischen Fasern dazu, zu sehr aneinander zu haften und die Geschmeidigkeit ist herabgesetzt. Hierdurch kommt es schneller zur Bildung von Mikrorissen und Brüchen.

In bestimmten Ausführungsformen übersteigt der Massenanteil des Alkylsilans und/oder PEG-Silans den Massenanteil des funktionellen Silans. In bestimmten Ausführungsformen übersteigt der Massenanteil des Alkylsilans und/oder PEG-Silans den Massenanteil des funktionellen Silans um einen Faktor von wenigstens 1,05, weiter bevorzugt 1,1, weiter bevorzugt 1,2, weiter bevorzugt 1,3, weiter bevorzugt 1,4, weiter bevorzugt 1,5, weiter bevorzugt 1,6. Bevorzugt übersteigt der Massenanteil des Alkylsilans und/oder PEG-Silans den Massenanteil des funktionellen Silans um einen Faktor von höchstens 100, weiter höchstens 80, weiter höchstens 60 weiter bevorzugt höchstens 40, weiter bevorzugt höchstens 30, weiter bevorzugt höchstens 20, weiter bevorzugt höchstens 10.

In einer anderen Ausführungsform übersteigt der Massenanteil des funktionellen Silans den Massenanteil des Alkylsilans und/oder PEG-Silans. In einer Ausführungsform übersteigt der Massenanteil des funktionellen Silans den Massenanteil des Alkylsilans und/oder PEG-Silans um einen Faktor von wenigstens 1,05, weiter bevorzugt 1,1, weiter bevorzugt 1,2, weiter bevorzugt 1,3, weiter bevorzugt 1,4, weiter bevorzugt 1,5, weiter bevorzugt 1,6. Bevorzugt übersteigt der Massenanteil des funktionellen Silans den Massenanteil des Alkylsilans und/oder PEG-Silans um einen Faktor von höchstens 100, weiter höchstens 80, weiter höchstens 60 weiter bevorzugt höchstens 40, weiter bevorzugt höchstens 30, weiter bevorzugt höchstens 20, weiter bevorzugt höchstens 10.

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | - |

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | - |
| PEG-Silan | 1-90 |

In einer weiteren Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | 1-90 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | - |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | - |
| PEG-Silan | 21-65 |

In einer weiteren Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | 21-65 |

Bevorzugt umfasst die Funktionsschicht alternativ oder zusätzlich zu wenigstens einem der Silane mindestens eine Fettsäure. Die Fettsäure kann eine gesättigte oder eine ungesättigte Fettsäure sein. In besonders bevorzugten Ausführungsformen ist die Fettsäure eine gesättigte Fettsäure. Die Fettsäure erhöht die mechanische Stabilität der optischen Faser und vermindert die Reibung zwischen den optischen Fasern. Außerdem kann die Fettsäure eine hydrophobe Schutzschicht um die Faser bilden.

Bevorzugt weist die Fettsäure eine Kettenlänge von mindestens 10 Kohlenstoffatomen auf, weiter bevorzugt von mindestens 11, weiter bevorzugt von mindestens 12, weiter bevorzugt von mindestens 13, weiter bevorzugt von mindestens 14, weiter bevorzugt von mindestens 15, weiter bevorzugt von mindestens 16. Bevorzugt weist die Fettsäure eine Kettenlänge von höchstens 40 Kohlenstoffatomen auf, weiter bevorzugt von höchstens 35, weiter bevorzugt von höchstens 30, weiter bevorzugt von höchstens 28, weiter bevorzugt von höchstens 26, weiter bevorzugt von höchstens 24, weiter bevorzugt von höchstens 22. In bevorzugten Ausführungsformen weist die Fettsäure eine Kettenlänge von 10 bis 40 Kohlenstoffatomen auf, weiter bevorzugt von 11 bis 35, weiter bevorzugt von 12 bis 30, weiter bevorzugt von 13 bis 28. In besonders bevorzugten Ausführungsformen weist die Fettsäure eine Kettenlänge von 14 bis 22 Kohlenstoffatomen auf. Ist die Kettenlänge zu klein, wird die Haftung der Funktionsschicht an der Oberfläche der Mantelschicht vermindert. Ist die Kettenlänge zu groß, neigen die optischen Fasern dazu, zu sehr aneinander zu haften.

Bevorzugt weist die Fettsäure einen Schmelzpunkt von mindestens 35°C auf, weiter bevorzugt von mindestens 39°C, weiter bevorzugt von mindestens 42°C, weiter bevorzugt von mindestens 48°C, weiter bevorzugt von mindestens 52°C, weiter bevorzugt von mindestens 54°C, weiter bevorzugt von mindestens 56°C, weiter bevorzugt von mindestens 58°C, weiter bevorzugt von mindestens 60°C, weiter bevorzugt von mindestens 65°C weiter bevorzugt von mindestens 70°C. Bevorzugt weist die Fettsäure einen Schmelzpunkt von höchstens 180°C auf, weiter bevorzugt von höchstens 160°C, weiter bevorzugt von höchstens 140°C, weiter bevorzugt von höchstens 120°C, weiter bevorzugt von höchstens 100°C, weiter bevorzugt von höchstens 95°C, weiter bevorzugt von höchstens 90°C. Bevorzugt weist die Fettsäure einen Schmelzpunkt von 35°C bis 180°C, von 39°C bis 160°C, oder von 42°C bis 100°C auf. In besonders bevorzugten Ausführungsformen weist die Fettsäure einen Schmelzpunkt von 53°C bis 90°C auf. Ist der Schmelzpunkt der Fettsäure zu niedrig, vermindert dies die Haftung der Funktionsschicht an der optischen Faser. Hierdurch kommt es zu einem verringerten Schutz der optischen Faser, beispielsweise vor Mikrorissen und Brüchen. Ist der Schmelzpunkt der Fettsäure zu hoch, kleben die optischen Fasern zu stark aneinander. Hierdurch wird die Geschmeidigkeit verringert und es kommt bei erhöhter und vermehrter Biegebelastung zu Mikrorissen und Brüchen. Bevorzugt weist die Fettsäure einen Siedepunkt von mindestens 200°C auf, weiter bevorzugt von mindestens 220°C, weiter bevorzugt von mindestens 240°C, weiter bevorzugt von mindestens 260°C, weiter bevorzugt von mindestens 280°C, weiter bevorzugt von mindestens 300°C. Bevorzugt weist die Fettsäure einen Siedepunkt von höchstens 500°C auf, weiter bevorzugt von höchstens 400°C, weiter bevorzugt von höchstens 380°C, weiter bevorzugt von höchstens 330°C. Bevorzugt weist die Fettsäure einen Siedepunkt von 200°C bis 500°C auf, von 220°C bis 400°C, oder von 240°C bis 380°C. Beispiele für erfindungsgemäße verwendbare Fettsäuren sind Caprinsäure, Laurinsäure, Myristinsäure, Palmitinsäure, Margarinsäure, Stearinsäure und Arachinsäure.

In bestimmten Ausführungsformen umfasst die Funktionsschicht eine Mehrzahl von Fettsäuren. In bestimmten Ausführungsformen umfasst die Funktionsschicht mindestens zwei Fettsäuren, mindestens drei Fettsäuren, oder mindestens vier Fettsäuren.

Bevorzugt weist die Mehrzahl von Fettsäuren einen Schmelzpunkt ihrer Mischung von mindestens 35°C auf, weiter bevorzugt von mindestens 39°C, weiter bevorzugt von mindestens 42°C, weiter bevorzugt von mindestens 48°C, weiter bevorzugt von mindestens 52°C, weiter bevorzugt von mindestens 54°C, weiter bevorzugt von mindestens 56°C, weiter bevorzugt von mindestens 58°C, weiter bevorzugt von mindestens 60°C, weiter bevorzugt von mindestens 65°C weiter bevorzugt von mindestens 70°C. Bevorzugt weist die Mehrzahl von Fettsäure in Form ihrer Mischung einen Schmelzpunkt von höchstens 180°C, weiter bevorzugt von höchstens 160°C, weiter bevorzugt von höchstens 140°C, weiter bevorzugt von höchstens 120°C, weiter bevorzugt von höchstens 100°C, weiter bevorzugt von höchstens 95°C, weiter bevorzugt von höchstens 90°C. Bevorzugt weist die Mehrzahl von Fettsäure als Mischung einen Schmelzpunkt von 35°C bis 180°C, von 39°C bis 160°C, oder von 42°C bis 140°C auf. Der Schmelzpunkt als Mischung meint den Schmelzpunkt den eine Mischung der die Mehrzahl von Fettsäuren bildenden einzelnen Fettsäuren in dem eingesetzten Verhältnis aufweist. Ist der Schmelzpunkt der Mehrzahl von Fettsäuren zu niedrig, vermindert dies die Haftung der Funktionsschicht an der optischen Faser. Hierdurch kommt es zu einem verringerten Schutz der optischen Faser, beispielsweise vor Mikrorissen und Brüchen. Ist der Schmelzpunkt der Mehrzahl von Fettsäuren zu hoch, kleben die optischen Fasern zu stark aneinander. Hierdurch wird die Geschmeidigkeit verringert und es kommt bei erhöhter und vermehrter Biegebelastung zu Mikrorissen und Brüchen.

Bevorzugt umfasst die Funktionsschicht eine oder mehrere Fettsäuren mit einem Anteil von mindestens 1 Gew.-%, weiter bevorzugt von mindestens 2 Gew.-%, weiter bevorzugt von mindestens 3 Gew.-%, weiter bevorzugt von mindestens 4 Gew.-%, weiter bevorzugt von mindestens 5 Gew.-%, weiter bevorzugt von mindestens 6 Gew.-%, weiter bevorzugt von mindestens 7 Gew.-%. Bevorzugt umfasst die Funktionsschicht mindestens eine Fettsäure mit einem Anteil von höchstens 85 Gew.-%, weiter bevorzugt von höchstens 80 Gew.-%, weiter bevorzugt von höchstens 75 Gew.-%, weiter bevorzugt von höchstens 70 Gew.-%, weiter bevorzugt von höchstens 65 Gew.-%, weiter bevorzugt von höchstens 60 Gew.-%, weiter bevorzugt von höchstens 56 Gew.-%. In bevorzugten Ausführungsformen umfasst die Funktionsschicht mindestens eine Fettsäure mit einem Anteil von 1-85 Gew.-%, weiter bevorzugt von 2-80 Gew.-%, weiter bevorzugt von 3-75 Gew.-%, weiter bevorzugt von 4-70 Gew.-%. In einer besonders bevorzugten Ausführungsform umfasst die Funktionsschicht eine oder mehrere Fettsäuren mit einem Anteil von insgesamt 7 bis 56 Gew.-%. Ist der Anteil der mindestens einen Fettsäure zu niedrig, gleiten die optischen Fasern schlechter aneinander. Ist der Anteil der mindestens einen Fettsäure zu hoch, verkleben die optischen Fasern miteinander.

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | - |
| PEG-Silan | - |
| Fettsäure | 1-85 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | - |
| PEG-Silan | - |
| Fettsäure | 7-56 |

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | - |
| Fettsäure | 1-85 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | - |
| Fettsäure | 7-56 |

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | 1-90 |
| Fettsäure | 1-85 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | 21-65 |
| Fettsäure | 7-56 |

In Ausführungsformen umfasst die Funktionsschicht alternativ oder zusätzlich zu wenigstens einem der Silane mindestens einen polyvalenten Alkohol. Erfindungsgemäße polyvalente Alkohole sind Glycerol, Diethylenglykol oder 1,5-Pentandiol sowie Alkanpolyole, wie Alkandiole und Alkantriole, insbesondere Alkanpolyole mit Kettenlängen von 2 bis 10 Kohlenstoffatomen, bevorzugt von 3 bis 8 Kohlenstoffatomen. Bevorzugt umfasst die Funktionsschicht mindestens einen polyvalenten Alkohol mit einem Anteil von mindestens 3 Gew.-%, weiter bevorzugt von mindestens 5 Gew.-%, weiter bevorzugt von mindestens 10 Gew.-%, weiter bevorzugt von mindestens 15 Gew.-%, weiter bevorzugt von mindestens 20 Gew.-%, weiter bevorzugt von mindestens 25 Gew.-%, weiter bevorzugt von mindestens 30 Gew.-%. Bevorzugt umfasst die Funktionsschicht mindestens einen polyvalenten Alkohol mit einem Anteil von höchstens 90 Gew.-%, weiter bevorzugt von höchstens 87 Gew.-%, weiter bevorzugt von höchstens 84 Gew.-%, weiter bevorzugt von höchstens 81 Gew.-%, weiter bevorzugt von höchstens 79 Gew.-%, weiter bevorzugt von höchstens 77 Gew.-%, weiter bevorzugt von höchstens 75 Gew.-%. In bevorzugten Ausführungsformen umfasst die Funktionsschicht den polyvalenten Alkohol mit einem Anteil von 3 bis 90 Gew.-%, weiter bevorzugt von 5 bis 87 Gew.-%, weiter bevorzugt von 10 bis 84 Gew.-%, weiter bevorzugt von 15 bis 81 Gew.-%. In besonders bevorzugten Ausführungsformen umfasst die Funktionsschicht den polyvalenten Alkohol mit einem Anteil von 30 bis 75 Gew.-%. Der polyvalente Alkohol verbessert das Aneinandergleiten von optischen Fasern und vermindert damit das Auftreten von Mikrorissen und Brüchen. Der polyvalente Alkohol verbessert die Händelbarkeit, die Packungsdichte und Wiedervereinzelbarkeit der optischen Faser beispielsweise in einem optischen Faserbündel, insbesondere führt der polyvalente Alkohol zu einer erwünschten "wet behaviour", also der Eigenschaft eines Faserartikels, sich wie ein nasser Faserartikel zu verhalten.

Die Funktionsschicht kann alternativ oder zusätzlich zu wenigstens einem der Silane mindestens ein Polyalkylenoxid umfassen. Erfindungsgemäße Polyalkylenoxide sind insbesondere Polyglykole wie beispielsweise Polyethylenglykol und Polypropylenglykol sowie andere Polyalkylenoxide aus Monomeren mit 2 bis 6 Kohlenstoffatomen. Bevorzugt umfasst die Funktionsschicht mindestens ein Polyalkylenoxid mit einem Anteil von mindestens 2 Gew.-%, weiter bevorzugt von mindestens 3 Gew.-%, weiter bevorzugt von mindestens 4 Gew.-%, weiter bevorzugt von mindestens 5 Gew.-%, weiter bevorzugt von mindestens 6 Gew.-%, weiter bevorzugt von mindestens 7 Gew.-%, weiter bevorzugt von mindestens 8 Gew.-%. Bevorzugt umfasst die Funktionsschicht mindestens ein Polyalkylenoxid mit einem Anteil von höchstens 90 Gew.-%, weiter bevorzugt von höchstens 85 Gew.-%, weiter bevorzugt von höchstens 80 Gew.-%, weiter bevorzugt von höchstens 75 Gew.-%, weiter bevorzugt von höchstens 70 Gew.-%, weiter bevorzugt von höchstens 68 Gew.-%, weiter bevorzugt von höchstens 65 Gew.-%. In bevorzugten Ausführungsformen umfasst die Funktionsschicht mindestens ein Polyalkylenoxid mit einem Anteil von 1 bis 90 Gew.-%, weiter bevorzugt von 2 bis 85 Gew.-%, weiter bevorzugt von 3 bis 80 Gew.-%, weiter bevorzugt von 4 bis 75 Gew.-%. In einer besonders bevorzugten Ausführungsformen umfasst die Funktionsschicht das Polyalkylenoxid mit einem Anteil von 8 bis 65 Gew.-%. Das Polyalkylenoxid vermindert die Reibung zwischen den optischen Fasern und verbessert die mechanische Stabilität der optischen Fasern. Das Polyalkylenoxid verbessert weiterhin den Zusammenhalt von optischen Fasern beispielsweise in einem optischen Faserbündel.

In bevorzugten Ausführungsformen hat das Polyalkylenoxid eine Kettenlänge von mindestens 3, weiter bevorzugt von mindestens 4, weiter bevorzugt mindestens von 5, weiter bevorzugt von mindestens 6, weiter bevorzugt von mindestens 7, weiter bevorzugt von mindestens 8 Alkylenoxideinheiten auf. Bevorzugt weist das Polyalkylenoxid eine Kettenlänge von höchstens 100, weiter bevorzugt von höchstens 75, weiter bevorzugt von höchstens 50, weiter bevorzugt von höchstens 20, weiter bevorzugt von höchstens 14 Alkylenoxideinheiten auf. In Ausführungsformen weist das Polyalkylenoxid eine Kettenlänge von 3 bis 100, weiter bevorzugt von 4 bis 50, weiter bevorzugt von 6 bis 20 Alkylenoxideinheiten auf. In besonders bevorzugten Ausführungsformen weist das Polyalkylenoxid eine Kettenlänge von 7 bis 14 Alkylenoxideinheiten auf. Es ist vorteilhaft, Polyalkylenoxide einzusetzen, die bei Raumtemperatur (20°C, 1013 hPa) flüssig sind.

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | - |
| Fettsäure | - |
| Polyvalenter Alkohol | - |
| Polyalkylenoxid | -90 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | - |
| Fettsäure | - |
| Polyvalenter Alkohol | - |
| Polyalkylenoxid | 8-65 |

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | - |
| Fettsäure | 1-85 |
| Polyvalenter Alkohol | - |
| Polyalkylenoxid | -90 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | - |
| Fettsäure | 7-56 |
| Polyvalenter Alkohol | - |
| Polyalkylenoxid | 8-65 |

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | 1-90 |
| Fettsäure | 1-85 |
| Polyvalenter Alkohol | 3-90 |
| Polyalkylenoxid | -90 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | 21-65 |
| Fettsäure | 7-56 |
| Polyvalenter Alkohol | 30-75 |
| Polyalkylenoxid | 8-65 |

In einer Ausführungsform umfasst die Funktionsschicht die folgenden Verbindungen:

| **Verbindung** | **Anteil (Gew.-%)** |
|---|---|
| Funktionelles Silan | 0,1-70 |
| Alkylsilan | 0,8-65 |
| PEG-Silan | 1-90 |
| Fettsäure | 1-85 |
| Polyvalenter Alkohol | 3-90 |
| Polyalkylenoxid | -90 |

In einer Ausführungsform umfasst die Funktionsschicht die folgenden Verbindungen:

| **Verbindung** | **Anteil (Gew.-%)** |
|---|---|
| Funktionelles Silan | 2-40 |
| Alkylsilan | 6-35 |
| PEG-Silan | 21-65 |
| Fettsäure | 7-56 |
| Polyvalenter Alkohol | 30-75 |
| Polyalkylenoxid | 8-65 |

In einer Ausführungsform umfasst die Funktionsschicht die folgenden Verbindungen:

| **Verbindung** | **Anteil (Gew.-%)** |
|---|---|
| Funktionelles Silan | 5-30 |
| Alkylsilan | 12-35 |
| Fettsäure | 15-56 |
| Polyalkylenoxid | 20-50 |

IR-Spektren von Funktionsschichten können aufgenommen werden, indem ein ATR-Kristall auf die Funktionsschicht gedrückt wird. Da die Spektren im ATR-Modus aufgenommen werden, entspricht das Spektrum einer Faser dem Spektrum der Funktionsschicht. Die Funktionsschicht weist eine charakteristische Absorption im Infrarot-Bereich im *Fourier-transform-Infrared-Attenuated-Total-Reflection* (FTIR-ATR)-Spektrum auf. Das IR-Spektrum der Funktionsschicht auf den Fasern der Faserartikel dieser Erfindung sind aufgrund der einzigartigen Zusammensetzung charakteristisch. Insbesondere unterscheiden sie sich von den IR-Spektren der Funktionsschichten im Stand der Technik. Beispielsweise weisen die Funktionsschichten dieser Erfindung - wenn überhaupt - nur sehr schwache Banden im Wellenzahlbereich um 1750 cm⁻¹ (±25 cm⁻¹) auf, was auf die bevorzugte Abwesenheit von Polyacrylaten und Polymethacrylaten zurückzuführen ist. Gleiches gilt für Banden im Bereich von 1590 cm⁻¹ (±25 cm⁻¹) auf, was auf die bevorzugte Abwesenheit von aromatischen Komponenten zurückzuführen ist. Viele aromatische Verbindungen sind krebserzeugend und daher nicht erwünscht.

Bevorzugt weist die Funktionsschicht im FTIR-ATR-Spektrum ein Verhältnis der maximalen Absorptionsbandenhöhe im Bereich von 3200 cm⁻¹ bis 3600 cm⁻¹ zu der maximalen Absorptionsbandenhöhe im Bereich von 3800 cm⁻¹ bis 4000 cm⁻¹ von mindestens 1,3 auf, weiter bevorzugt von mindestens 1,4, weiter bevorzugt von mindestens 1,5, weiter bevorzugt von mindestens 1,6, weiter bevorzugt von mindestens 1,7, weiter bevorzugt von mindestens 1,8, weiter bevorzugt von mindestens 1,9, weiter bevorzugt von mindestens 2,0, weiter bevorzugt von mindestens 2,1, weiter bevorzugt von mindestens 2,2. Bevorzugt weist die Funktionsschicht im FTIR-ATR-Spektrum ein Verhältnis der maximalen Absorptionsbandenhöhe im Bereich von 3200 cm⁻¹ bis 3600 cm⁻¹ zu der maximalen Absorptionsbandenhöhe im Bereich von 3800 cm⁻¹ bis 4000 cm⁻¹ von höchstens 35 auf, weiter bevorzugt von höchstens 30, weiter bevorzugt von höchstens 25, weiter bevorzugt von höchstens 20, weiter bevorzugt von höchstens 15, weiter bevorzugt von höchstens 10, weiter bevorzugt von höchstens 9, weiter bevorzugt von höchstens 8, weiter bevorzugt von höchstens 7, weiter bevorzugt von höchstens 6. Bevorzugt weist die Funktionsschicht im FTIR-ATR-Spektrum ein Verhältnis der maximalen Absorptionsbandenhöhe im Bereich von 3200 cm⁻¹ bis 3600 cm⁻¹ zu der maximalen Absorptionsbandenhöhe im Bereich von 3800 cm⁻¹ bis 4000 cm⁻¹ von 1,3 bis 35 auf, weiter bevorzugt 1,4 bis 30, weiter bevorzugt 1,5 bis 25, weiter bevorzugt 1,6 bis 6. Die hier beschriebene Absorption wird beispielsweise durch ein vorteilhaftes Verhältnis des funktionellen Silans und/oder dem optionalen polyvalenten Alkohol zu den weiteren Komponenten der Funktionsschicht hervorgerufen. Ist die hier beschriebene relative Absorption von 3200 cm⁻¹ bis 3600 cm⁻¹ zu gering, ist das Verhältnis des funktionellen Silans zu den anderen Komponenten der Funktionsschicht zu klein. Hierdurch haftet die Funktionsschicht schlechter an der Oberfläche der Mantelschicht. Weiterhin ist Verklebbarkeit der Funktionsschicht mit weiteren Schichten wie beispielsweise einer Klebeschicht in einem Verbund von optischen Faserbündeln verringert. Ist die hier beschriebene relative Absorption von 3200 cm⁻¹ bis 3600 cm⁻¹ dagegen zu groß, ist das Verhältnis des funktionellen Silans zu den anderen Komponenten der Funktionsschicht zu hoch. Dies führt dazu, dass sich die Reibung zwischen den optischen Fasern erhöht und die Gleitfähigkeit herabgesetzt ist.

Bevorzugt weist die Funktionsschicht im FTIR-ATR-Spektrum ein Verhältnis der maximalen Absorptionsbandenhöhe im Bereich von 800 cm⁻¹ bis 1200 cm⁻¹ zu der maximalen Absorptionsbandenhöhe im Bereich von 1500 cm⁻¹ bis 1900 cm⁻¹ von mindestens 1,1 auf, weiter bevorzugt von mindestens 1,2, weiter bevorzugt von mindestens 1,3, weiter bevorzugt von mindestens 1,4, weiter bevorzugt von mindestens 1,5, weiter bevorzugt von mindestens 1,6, weiter bevorzugt von mindestens 1,7, weiter bevorzugt von mindestens 1,8, weiter bevorzugt von mindestens 1,9, weiter bevorzugt von mindestens 2,0. Bevorzugt weist die Funktionsschicht im FTIR-ATR-Spektrum ein Verhältnis der maximalen Absorptionsbandenhöhe im Bereich von 800 cm⁻¹ bis 1200 cm⁻¹ zu der maximalen Absorptionsbandenhöhe im Bereich von 1500 cm⁻¹ bis 1900 cm⁻¹ von höchstens 300 auf, weiter bevorzugt von höchstens 200, weiter bevorzugt von höchstens 100, weiter bevorzugt von höchstens 50, weiter bevorzugt von höchstens 30, weiter bevorzugt von höchstens 20, weiter bevorzugt von höchstens 10, weiter bevorzugt von höchstens 7, weiter bevorzugt von höchstens 6, weiter bevorzugt von höchstens 5. Bevorzugt weist die Funktionsschicht im FTIR-ATR-Spektrum ein Verhältnis der maximalen Absorptionsbandenhöhe im Bereich von 800 nm bis 1200 nm zu der maximalen Absorptionsbandenhöhe im Bereich von 1500 cm⁻¹ bis 1900 cm⁻¹ von 1,3 bis 300 auf, weiter bevorzugt 1,4 bis 250, weiter bevorzugt 1,5 bis 200, weiter bevorzugt 2,0 bis 10. Die hier beschriebene Absorption wird beispielsweise durch ein vorteilhaftes Verhältnis des funktionellen Silans, des optionalen Alkylsilans und/oder des optionalen PEG-Silans insbesondere zu unerwünschten fotopolymerisierbaren bzw. fotopolymerisierten Verbindungen hervorgerufen. Ist die hier beschriebene relative Absorption von800 cm⁻¹ bis 1200 cm⁻¹ zu gering, ist das Verhältnis an fotopolymerisierbaren bzw. fotopolymerisierten Verbindungen zu hoch. Solche Funktionsschichten sind gesundheitlich nicht unbedenklich.

Bevorzugt weist die Funktionsschicht im FTIR-ATR-Spektrum ein Verhältnis der maximalen Absorptionsbandenhöhe im Bereich von 2700 cm⁻¹ bis 3000 cm⁻¹ zu der maximalen Absorptionsbandenhöhe im Bereich von 1500 cm⁻¹ bis 1900 cm⁻¹ von mindestens 1,1 auf, weiter bevorzugt von mindestens 1,2, weiter bevorzugt von mindestens 1,3, weiter bevorzugt von mindestens 1,4, weiter bevorzugt von mindestens 1,5, weiter bevorzugt von mindestens 1,6, weiter bevorzugt von mindestens 1,7, weiter bevorzugt von mindestens 1,8, weiter bevorzugt von mindestens 1,9, weiter bevorzugt von mindestens 2,0. Bevorzugt weist die Funktionsschicht im FTIR-ATR-Spektrum ein Verhältnis der maximalen Absorptionsbandenhöhe im Bereich von 2700 cm⁻¹ bis 3000 cm⁻¹ zu der maximalen Absorptionsbandenhöhe im Bereich von 1500 cm⁻¹ bis 1900 cm⁻¹ von höchstens 300 auf, weiter bevorzugt von höchstens 200, weiter bevorzugt von höchstens 100, weiter bevorzugt von höchstens 50, weiter bevorzugt von höchstens 30, weiter bevorzugt von höchstens 20, weiter bevorzugt von höchstens 10, weiter bevorzugt von höchstens 7, weiter bevorzugt von höchstens 6, weiter bevorzugt von höchstens 5. Bevorzugt weist die Funktionsschicht im FTIR-ATR-Spektrum ein Verhältnis der maximalen Absorptionsbandenhöhe im Bereich von 2700 cm⁻¹ bis 3000 cm⁻¹ zu der maximalen Absorptionsbandenhöhe im Bereich von 1500 und 1900 nm von 1,3 bis 300 auf, weiter bevorzugt 1,4 bis 250, weiter bevorzugt 1,5 bis 200, weiter bevorzugt 2,0 bis 5. Die hier beschriebene Absorption wird beispielsweise durch ein vorteilhaftes Verhältnis des funktionellen Silans, des optionalen Alkylsilans, des optionalen PEG-Silans und/oder der optionalen Fettsäure insbesondere zu unerwünschten fotopolymerisierbaren bzw. fotopolymerisierten Verbindungen hervorgerufen. Ist die hier beschriebene relative Absorption von 2700 cm⁻¹ bis 3000 cm⁻¹ zu gering, ist das Verhältnis an fotopolymerisierbaren bzw. fotopolymerisierten Verbindungen zu hoch. Solche Funktionsschichten sind gesundheitlich nicht unbedenklich und können dem Patienten gesundheitlichen Schaden zufügen.

Bevorzugt weist die Funktionsschicht im FTIR-ATR-Spektrum ein Verhältnis der maximalen Absorptionsbandenhöhe im Bereich von 3200 cm⁻¹ bis 3600 cm⁻¹ zu der maximalen Absorptionsbandenhöhe im Bereich von 1500 cm⁻¹ bis 1900 cm⁻¹ von mindestens 1,1 auf, weiter bevorzugt von mindestens 1,2, weiter bevorzugt von mindestens 1,3, weiter bevorzugt von mindestens 1,4, weiter bevorzugt von mindestens 1,5. Bevorzugt weist die Funktionsschicht im FTIR-ATR-Spektrum ein Verhältnis der maximalen Absorptionsbandenhöhe im Bereich von 3200 cm⁻¹ bis 3600 cm⁻¹ zu der maximalen Absorptionsbandenhöhe im Bereich von 1500 bis 1900 nm von höchstens 3,0 auf, weiter bevorzugt von höchstens 2,5, weiter bevorzugt von höchstens 2,2, weiter bevorzugt von höchstens 2,0. Bevorzugt die Funktionsschicht im FTIR-ATR-Spektrum ein Verhältnis der maximalen Absorptionsbandenhöhe im Bereich von 3200 cm⁻¹ bis 3600 cm⁻¹ zu der maximalen Absorptionsbandenhöhe im Bereich von 1500 cm⁻¹ bis 1900 cm⁻¹ von 1,1 bis 3 auf, weiter bevorzugt 1,1 bis 2,5, weiter bevorzugt 1,1 bis 2,0, oder 1,2 bis 2,5. Die hier beschriebene Absorption wird beispielsweise durch ein vorteilhaftes Verhältnis des funktionellen Silans und/oder dem optionalen polyvalenten Alkohol insbesondere zu unerwünschten fotopolymerisierbaren bzw. fotopolymerisierten Verbindungen hervorgerufen. Ist die hier beschriebene relative Absorption von 3200 cm⁻¹ bis 3600 cm⁻¹ zu gering, ist das Verhältnis an fotopolymerisierbaren bzw. fotopolymerisierten Verbindungen zu hoch. Solche Funktionsschichten sind gesundheitlich nicht unbedenklich.

Bevorzugt weist die Funktionsschicht im FTIR-ATR-Spektrum ein Verhältnis der maximalen Absorptionsbandenhöhe im Bereich von 800 cm⁻¹ bis 1200 cm⁻¹ zu der maximalen Absorptionsbandenhöhe im Bereich von 2700 cm⁻¹ bis 3000 cm⁻¹ von mindestens 1,1 auf, weiter bevorzugt von mindestens 1,2, weiter bevorzugt von mindestens 1,3, weiter bevorzugt von mindestens 1,4. Bevorzugt weist die Funktionsschicht im FTIR-ATR-Spektrum ein Verhältnis der maximalen Absorptionsbandenhöhe im Bereich von 800 cm⁻¹ bis 1200 cm⁻¹ zu der maximalen Absorptionsbandenhöhe im Bereich von 3200 cm⁻¹ bis 3600 cm⁻¹ von höchstens 35 auf, weiter bevorzugt von höchstens 30, weiter bevorzugt von höchstens 25, weiter bevorzugt von höchstens 20, weiter bevorzugt von höchstens 15, weiter bevorzugt von höchstens 10, weiter bevorzugt von höchstens 9,0, weiter bevorzugt von höchstens 5, weiter bevorzugt von höchstens 3,0, weiter bevorzugt von höchstens 2,0. Bevorzugt weist die Funktionsschicht im FTIR-ATR-Spektrum ein Verhältnis der maximalen Absorptionsbandenhöhe im Bereich von 800 cm⁻¹ bis 1200 cm⁻¹ zu der maximalen Absorptionsbandenhöhe im Bereich von 3200 cm⁻¹ bis 3600 cm⁻¹ von 1,1 bis 35 auf, weiter bevorzugt 1,2 bis 15, weiter bevorzugt 1,3 bis 2,0. Die hier beschriebene Absorption wird beispielsweise durch ein vorteilhaftes Verhältnis des funktionellen Silans und/oder des optionalen polyvalenten Alkohols insbesondere zu funktionellem Silan, optionalem Alkylsilans und/oder optionalen PEG-Silans hervorgerufen. Ist die hier beschriebene relative Absorption von 800 nm bis 1200 nm zu gering, ist das Verhältnis von funktionellem Silan zu gering. Ist die hier beschriebene relative Absorption von 800 cm⁻¹ bis 1200 cm⁻¹ zu hoch, ist das Verhältnis von funktionellem Silan zu hoch.

Für eine gute Gleitwirkung zwischen optischen Fasern, ohne dass diese miteinander verkleben, sollte die Funktionsschicht geeignete Reibungskoeffizienten aufweisen. Der Reibungskoeffizient kann nach einem dem Fachmann bekannten Verfahren an der Oberfläche der Funktionsschicht gemessen werden. Die Messung kann beispielsweise erfolgen, indem ein optischer Faserartikel oder entsprechender Glasstab aus gleichem Material wie der Faserartikel (Prüfartikel) in einem Winkel von 90° zu einem Reibpartner, der ebenfalls aus einem optischen Faserartikel oder entsprechenden Glasstab gebildet ist, mit einer Normalkraft Fn von 0,5 N mit einer Geschwindigkeit von 10 mm/min verschoben wird. Die Verschiebung erfolgt in longitudinaler Richtung, bezogen auf den Prüfarti-kel. Der Reibpartner ist ebenfalls aus einem optischen Faserartikel oder entsprechenden Glasstab gebildet. Dabei entsteht je nach Reibwert eine Reibkraft, die als Maß für den Reibwert (Reibwert = Quotient aus Reibkraft und Normalkraft) ermittelt wird. Bevorzugt weist die Funktionsschicht einen Reibungskoeffizienten von höchstens 0,5 auf, weiter bevorzugt von höchstens 0,45, weiter bevorzugt von höchstens 0,35, weiter bevorzugt von höchstens 0,3, weiter bevorzugt von höchstens 0,2, weiter bevorzugt von höchstens 0,15. Bevorzugt weist die Funktionsschicht einen Reibungskoeffizienten von mindestens 0,001 auf, weiter bevorzugt von mindestens 0,01, weiter bevorzugt von mindestens 0,03, weiter bevorzugt von mindestens 0,05. In bevorzugten Ausführungsformen weist die Funktionsschicht einen Reibungskoeffizienten von 0,001 bis 0,5 auf, weiter bevorzugt von 0,01 bis 0,35, weiter bevorzugt von 0,03 bis 0,2. In besonders bevorzugten Ausführungsformen weist die Funktionsschicht einen Reibungskoeffizienten von weniger als 0,2 auf. Ist der Reibungskoeffizient zu hoch, gleiten die optischen Fasern schlechter aneinander und es kommt zu Mikrorissen und Brüchen. Ist der Reibungskoeffizient zu niedrig, halten die optischen Fasern beispielsweise in einem optischen Faserbündel schlechter zusammen.

Die Oberfläche der Funktionsschicht sollte einen geeigneten Kontaktwinkel aufweisen, um beispielsweise eine gute Benetzbarkeit der optischen Faser beispielsweise mit einem Klebstoff in einem Verbund aus optischen Faserbündeln zu erreichen. Der Kontaktwinkel kann an der Oberfläche der Funktionsschicht nach einem dem Fachmann bekannten Verfahren gemessen werden (beispielsweise nach ISO DIN 55660-2:2011). Bevorzugt weist die Funktionsschicht einen Kontaktwinkel von mindestens 10° auf, weiter bevorzugt von mindestens 20°, weiter bevorzugt von mindestens 40°, weiter bevorzugt von mindestens 60°, weiter bevorzugt von mindestens 80°. Bevorzugt weist die Funktionsschicht einen Kontaktwinkel von höchstens 125° auf, weiter bevorzugt von höchstens 100°, In bevorzugten Ausführungsformen weist die Funktionsschicht einen Kontaktwinkel von 20° bis 120° auf, weiter bevorzugt von 40° bis 100°, weiter bevorzugt von 60° bis 100°. In besonders bevorzugten Ausführungsformen weist die Funktionsschicht einen Kontaktwinkel von 50° bis 85° auf. Ist der Kontaktwinkel zu klein, ist die Oberfläche der Funktionsschicht zu hydrophil und führt zu einer verminderten chemischen Schutzwirkung. Ist der Kontaktwinkel zu groß, ist die Benetzbarkeit der Oberfläche der Funktionsschicht beispielsweise mit einer Klebeschicht in einem Verbund vermindert.

Die Funktionsschicht sollte gut an der Oberfläche der Faser haften, eine gute aber begrenzte Haftung der Fasern untereinander ermöglichen und eine gute Verklebung eines Faserartikels, insbesondere Faserbündels, an anderen Materialien, z.B. einer Hülse, ermöglichen. Das im nachfolgend beschriebenen Ausdrücktest erzielte Ergebnis ist ein Maß für die Haftung der Fasern aneinander. Im Ausdrücktest wird ein Faserbündel oder zum Vergleich mehrere, ggf. unterschiedliche, Faserbündel mit einem Durchmesser von 5 mm mit identischem Kleber in je eine Edelstahlhülse geklebt, beispielsweise mit einem Zwei-Komponenten-Epoxydharz-Klebstoff, bevorzugt lösemittelfrei und raumtemperaturvernetzend (z.B. Araldit AY103-1 Härter; REN HY 956, Fa. Huntsman; Aushärtung bei 60°C für 2h). Die Endfläche der Faserbündel wird geschliffen und poliert. Mit einem Prüfdorn mit rundem Querschnitt, einem Durchmesser von 2,5 mm und planer Endfläche werden anschließend die Ausdrückkräfte, zumindest qualitativ bzw. relativ zueinander, bestimmt, indem der Prüfdorn in Faserrichtung auf die in der Hülse verklebten Fasern gedrückt wird. Die Ausdrückgeschwindigkeit beträgt 6 mm/min. Es treten zwei Versagensbilder auf. Im ersten Fall versagt die Verklebung von Faserbündel zu Edelstahlhülse (gesamtes Faserbündel wird aus Hülse geschoben). Im zweiten Fall versagt die Faserverklebung untereinander. Im ersten Fall ist die Haftung der Fasern untereinander besser als die Haftung des Faserbündels an der Edelstahlhülse. Im zweiten Fall deutet dies auf eine mangelhafte Adhäsion des Klebstoffes auf der Glasfaser hin, was eine unzureichende Haftvermittlung der Funktionsschicht anzeigt. Die detektierte maximale Kraft, die benötigt wird, um den unter dem Prüfdorn befindliche verklebte Faserbündel gegenüber der Edelstahlhülse, bzw. den umliegenden Fasern zu verschieben, ist die Ausdrückkraft.

Bevorzugt weist der optische Faserartikel eine Ausdrückkraft von mindestens 250 N, weiter bevorzugt von mindestens 300 N, weiter bevorzugt von mindestens 350 N, weiter bevorzugt von mindestens 400 N, weiter bevorzugt von mindestens 500 N, mindestens 700 N oder mindestens 900 N. In Ausführungsformen weist der optische Faserartikel eine Ausdrückkraft von höchstens 5.000 N auf, weiter bevorzugt von höchstens 4.500 N, weiter bevorzugt von höchstens 4.000 N, weiter bevorzugt von höchstens 3.500 N, weiter bevorzugt von höchstens 3.000 N. In bevorzugten Ausführungsformen weist der optische Faserartikel eine Ausdrückkraft von 100 bis 5.000 N auf, weiter bevorzugt von 150 bis 4.500 N, weiter bevorzugt von 200 bis 4.000 N. Ist die Ausdrückkraft zu gering, haftet die Funktionsschicht schlecht an der Oberfläche der Mantelschicht oder die Haftvermittlung der Funktionsschicht ist zu schwach ausgeprägt.

Bevorzugt weist der optische Faserartikel Fasern auf, die eine hohe Bruchlänge, insbesondere von wenigstens 20 m, aufweisen. Weiter bevorzugt weisen die Fasern eine Bruchlänge von mindestens 30 m auf, weiter bevorzugt von mindestens 40 m, weiter bevorzugt von mindestens 50 m, weiter bevorzugt von mindestens 60 m, weiter bevorzugt von mindestens 70 m, weiter bevorzugt von mindestens 80 m, weiter bevorzugt von mindestens 90 m. Ist die Länge zu klein, brechen die optischen Fasern zu häufig und sind für den Einsatz als Lichtleiter und/oder Bildleiter ungeeignet. Weiterhin geht von gebrochenen optischen Fasern eine Gefahr für die Gesundheit aus. Die Bruchlänge kann wie folgt gemessen werden: Die Prüffaser wird als Endlosfaser einer Länge von mindestens 2 km auf einer Spule (z. B. Standard-Styroporspule mit Umfang 0,49 m) bereitgestellt. Eine Zug-Biege-Vorrichtung unterwirft die zu prüfende Faser einer definierten Biegebelastung, in dem die Faser über 4 Rollen mit dem Radius 6 mm umgespult wird. Die Prüffaser, die dabei unter einer konstanten Spannung steht, wird von einer gebremsten Spule abgewickelt und auf eine zweite motorisch angetriebene Spule aufgewickelt. Tritt ein Faserbruch auf, so wird der Abspulvorgang über einen Bruchsensor gestoppt. Die Faser wird zur Fortsetzung der Messung neu aufgelegt. Die Bruchlänge ist die Faserlänge, bei der die Bruchwahrscheinlichkeit 63 % beträgt (Weibull-Verteilung). Zur Berechnung werden vorzugsweise wenigstens 10 Messungen, vorzugsweise 25 Messungen durchgeführt. Die Prüfung wird bei einer Zugkraft von 25+/-1 cN und einer Umspulgeschwindigkeit von 25+/- 1 U./min durchgeführt (entspricht 12,5 m/min).

Der optische Faserartikel sollte Fasern enthalten, die eine hohe mechanische Stabilität aufweisen und möglichst nicht brechen. Die Bruchneigung der Fasern kann im Bruchschlingentest gemäß DIN 58141-6:2011 gemessen werden. Der Test dient zur Ermittlung der Grundfestigkeit eines Faserartikels. Mit dem Verfahren wird der mechanische Biegeradius von Lichtleitfasern dadurch bestimmt, dass diese mittels einer enger werdenden 360°-Schlinge bis zum Faserbruch belastet werden (zerstörende Prüfung). Ergebnis des Bruchschlingentests ist ein Biegeradius, also ein Maß dafür, wie weit die Faser gebogen werden kann, ohne zu brechen. Die Fasern des optischen Faserartikels weisen vorzugsweise einen Biegeradius von weniger als 10 mm, insbesondere weniger als 5 mm, weniger als 3 mm oder sogar weniger als 2 mm auf. Die Fasern weisen insbesondere auch nach hydrolytischem Stress weiterhin einen sehr kleinen Biegeradius auf. So bleibt der Biegeradius der Fasern vorzugsweise auch nach Lagerung der Fasern in Wasser bei 50°C für 5 Tage bei weniger als 12 mm, weniger als 8 mm oder weniger als 6 mm. Dass die Fasern auch bei hydrolytischem Stress ihre guten Biegeeigenschaften weitgehend behalten, ist ein großer Vorteil hinsichtlich der Reinigung und Sterilisation von optischen Faserartikeln.

Acrylate, Methacrylate, Vinyl- und Styrenverbindungen sowie andere ungesättigte Verbindungen, die bei UV-Bestrahlung aushärten, sind potenziell schädlich für die Umwelt und können gesundheitlichen Schaden am Menschen anrichten. Die Funktionsschicht sollte daher einen möglichst geringen Anteil an fotopolymerisierbaren und/oder fotopolymerisierten Substanzen aufweisen.

Bevorzugt weist der optische Faserartikel einen Gehalt an fotopolymerisierten Polymeren in der Funktionsschicht bezogen auf die Masse der Funktionsschicht von weniger als 1 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%. In besonders bevorzugten Ausführungsformen ist die Funktionsschicht frei von fotopolymerisierten Polymeren. "Fotopolymerisierte Polymere" meint Polymere, die durch Polymerisation UV-härtbarer Mono- oder Oligomere herstellbar sind. Erfindungsgemäß bevorzugt vermiedene fotopolymerisierte Polymere sind beispielsweise Polyacrylate, Polymethacrylate, Polyvinylpolymere, Polystyrol und/oder Derivate davon. Ist der Anteil an fotopolymerisierten Polymeren in der Funktionsschicht zu hoch, gehen von dem optischen Faserartikel potenziell gesundheitliche Risiken aus. Insbesondere bei medizinischen Geräten, welche häufig autoklaviert werden, kann es vorkommen, dass fotopolymerisierte Polymere Restmonomere oder Fotoinitiatoren freisetzen. Insbesondere bei medizinischen Geräten, die mit menschlichen Organen in Kontakt kommen, wie beispielsweise Endoskopen, soll daher der Anteil an fotopolymerisierten Polymeren nicht höher als hier beschrieben sein. Insbesondere ist auch der Gehalt an fotopolymerisierbaren Mono- und/oder Oligomeren in der Funktionsschicht gering. Insbesondere ist der Gehalt an Acrylaten, Methacrylaten, Styren- und Vinylverbindungen in der Funktionsschicht vorzugsweise auf weniger als 1 Gew.-%, weniger als 0,1 Gew.-% oder weniger als 0,01 Gew.-% beschränkt.

Fotoinitiatoren sind möglicherweise schädlich für die menschliche Gesundheit. In bevorzugten Ausführungsformen, weist der optische Faserartikel einen Gehalt an Fotoinitiatoren und/oder Derivaten davon in der Funktionsschicht bezogen auf die Masse der Funktionsschicht von weniger als 5 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, noch weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%. In besonders bevorzugten Ausführungsformen ist die Funktionsschicht frei von Fotoinitiatoren und/oder Derivaten davon. "Fotoinitiatoren und/oder Derivate davon" meint chemische Verbindungen, die nach Absorption von Photonen wie beispielsweise UV-Licht reaktive Spezies bilden, die eine Reaktion wie beispielsweise eine Polymerisation starten können. Fotoinitiatoren und/oder Derivate davon können auch die bereits abreagierten Verbindungen davon meinen. Erfindungsgemäße Fotoinitiatoren sind beispielsweise radikalische Fotoinitatoren, kationische Fotoinitiatoren und/oder thermolatente Fotoinitiatoren. Solche Fotoinitiatoren sind beispielsweise Acylphosphinoxid, alpha-Alkoxy-Arylketon oder Aryldiazonium oder Kombinationen davon. Ist der Anteil an Fotoinitiatoren und/oder Derivaten davon in der Funktionsschicht zu hoch, gehen von dem optischen Faserartikel potenziell gesundheitliche Risiken aus. Dies ist insbesondere bei medizinischen Geräten unerwünscht.

Die Funktionsschicht sollte gut getrocknet sein und einen geringen Wassergehalt aufweisen. Bevorzugt weist der optische Faserartikel einen Restgehalt an Wasser in der Funktionsschicht bezogen auf die Masse der Funktionsschicht von weniger als 5 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, noch weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%. In besonders bevorzugten Ausführungsformen ist die Funktionsschicht frei von Wasser. Ist der Anteil an Wasser zu hoch, wird die isolierende Schutzwirkung der Funktionsschicht vermindert und die mechanische und chemische Stabilität des optischen Faserartikels wird vermindert.

Die Funktionsschicht sollte gut getrocknet sein und geringen Restgehalt wassermischbaren Lösungsmitteln aufweisen. Derartige Lösungsmittel sind insbesondere aliphatische Ether oder Alkohole mit bis zu 10 Kohlenstoffatomen und/oder Carbonsäuren mit bis zu 6 Kohlenstoffatomen. Beispiele für erfindungsgemäße wassermischbare Lösungsmittel sind Essigsäure, Ethanol, Isopropanol, Dipropyleneglycolmonomethylether oder Tripropyleneglycolmonomethylether. Bevorzugt weist der optische Faserartikel einen Restgehalt an wassermischbaren Lösungsmitteln in der Funktionsschicht bezogen auf die Masse der Funktionsschicht von weniger als 5 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, noch weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%. In besonders bevorzugten Ausführungsformen ist die Funktionsschicht frei von wassermischbaren Lösungsmitteln. Ist der Anteil an wassermischbaren Lösungsmitteln zu hoch, wird die isolierende Schutzwirkung der Funktionsschicht vermindert und die mechanische und chemische Stabilität des optischen Faserartikels wird vermindert.

In besonders bevorzugten Ausführungsformen weist der optische Faserartikel einen Restgehalt an kurzkettigen Carbonsäuren mit bis zu 10 Kohlenstoffatomen in der Funktionsschicht bezogen auf die Masse der Funktionsschicht von weniger als 5 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, noch weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%. Derartige kurzkettige Carbonsäuren sind beispielsweise Essigsäure oder Citronensäure. In besonders bevorzugten Ausführungsformen ist die Funktionsschicht frei von kurzkettigen Carbonsäuren mit bis zu 10 Kohlenstoffatomen.

In besonders bevorzugten Ausführungsformen weist der optische Faserartikel einen Restgehalt an aliphatischen Alkoholen mit bis zu vier Kohlenstoffatomen in der Funktionsschicht bezogen auf die Masse der Funktionsschicht von weniger als 5 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, noch weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%. Derartige kurzkettige Carbonsäuren sind beispielsweise Ethanol oder Isopropanol. In besonders bevorzugten Ausführungsformen ist die Funktionsschicht frei von aliphatischen Alkoholen mit bis zu vier Kohlenstoffatomen.

Der optische Faserartikel sollte gut geeignet für die Anwendung in medizinischen Geräten wie beispielsweise Endoskopen sein. Für die medizinische Anwendung sollte der optische Faserartikel möglichst beständig auch bei häufigem Autoklavieren sein. Hierfür ist es wichtig, dass der optische Faserartikel möglichst wenige Halogene aufweist. Bevorzugt weist der optische Faserartikel einen Gehalt an Halogenen in der Funktionsschicht bezogen auf die Funktionsschicht von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist die Funktionsschicht frei von Halogen. Halogene sind beispielsweise Chlor, Fluor, Brom und/oder Iod bzw. deren Anionen. Der Begriff "Halogen" umfasst insbesondere die Halogenide, vorzugsweise auch andere Halogenverbindungen. Eine zu hohe Konzentration an Halogenen in der Funktionsschicht führt zur Bildung der entsprechenden Halogensäuren, insbesondere beispielsweise bei der Dampfsterilisation. Die entsprechenden Halogensäuren können die Beständigkeit des optischen Faserartikels mindern sowie aus diesem austreten. Insbesondere greifen die Halogensäuren Materialien wie beispielsweise Edelstahl von Autoklaven und Endoskopen an und führen zur Bildung von unerwünschtem Rost.

Insbesondere kann ein Restgehalt an Chlorid in der Funktionsschicht zur Bildung von Salzsäure führen. Daher sollte der optische Faserartikel möglichst wenig Chlorid aufweisen. Bevorzugt weist der optische Faserartikel einen Gehalt an Chlorid in der Funktionsschicht bezogen auf die Funktionsschicht von weniger als 500 ppm (m/m) auf, weiter bevorzugt von weniger als 400 ppm (m/m), weiter bevorzugt von weniger als 300 ppm (m/m), weiter bevorzugt von weniger als 250 ppm (m/m), weiter bevorzugt von weniger als 200 ppm (m/m), weiter bevorzugt von weniger als 150 ppm (m/m), weiter bevorzugt von weniger als 100 ppm (m/m), weiter bevorzugt von weniger als 80 ppm (m/m), weiter bevorzugt von weniger als 60 ppm (m/m), weiter bevorzugt von weniger als 40 ppm (m/m), weiter bevorzugt von weniger als 20 ppm (m/m), noch weiter bevorzugt von weniger als 10 ppm (m/m). In besonders bevorzugten Ausführungsformen ist die Funktionsschicht frei von Chlorid. Ein zu hoher Gehalt an Chlorid mindert die Korrosionsbeständigkeit des optischen Faserartikels.

Für die Eignung des optischen Glasfaserartikels in der medizinischen bzw. diagnostischen Anwendung sollte der optische Glasfaserartikel biokompatibel sein. Bevorzugt ist der Faserartikel biokompatibel nach ISO10993-1:2018. Bevorzugt ist der optische Faserartikel biokompatibel im Zytotoxizitätstest nach ISO10993-5:2009.

Bevorzugt ist der optische Faserartikel biokompatibel nach ISO10993-1:2018 und/oder ISO10993-5:2009. Bevorzugt ist der optische Faserartikel biokompatibel nach USP Class VI.

Der optische Faserartikel sollte nur Verbindungen umfassen, die möglichst keine Gefahren für die Gesundheit von Menschen umfassen. Beispielsweise kommen auch Menschen bei der Herstellung und Verarbeitung des optischen Faserartikels mit diesem in Kontakt. Bevorzugt weist der optische Faserartikel einen Anteil an Verbindungen mit einer maximalen Arbeitsplatz-Konzentration (MAK-Wert) von weniger als 240 mg*m⁻³ in der Funktionsschicht bezogen auf die Masse der Funktionsschicht von weniger als 5 Gew.-% auf, weiter bevorzugt von weniger als 4 Gew.-%, weiter bevorzugt von weniger als 3 Gew.-%, weiter bevorzugt von weniger als 2 Gew.-%, weiter bevorzugt von weniger als 1 Gew.-%, weiter bevorzugt von weniger als 0,8 Gew.-%, weiter bevorzugt von weniger als 0,6 Gew.-%, weiter bevorzugt von weniger als 0,4 Gew.-%, noch weiter bevorzugt von weniger als 0,2 Gew.-%, weiter bevorzugt von weniger als 0,1 Gew.-%, weiter bevorzugt von weniger als 0,05 Gew.-%, weiter bevorzugt von weniger als 0,01 Gew.-%, weiter bevorzugt von weniger als 0,005 Gew.-%, weiter bevorzugt von weniger als 0,001 Gew.-%.

Bevorzugt umfasst die optische Faser einen Faserkern und einen Mantel bzw. eine Mantelschicht, die Funktionsschicht ist dann auf der Mantelschicht angeordnet. In bevorzugten Ausführungsformen besteht die Kernschicht aus einem Kernglas. In anderen Ausführungsformen besteht der Faserkern aus einem Kernpolymer. Geeignete Kernpolymere sind beispielsweise Polyacrylat, Polymethacrylat, Polyurethan, Polyester, Polyamid, und Mischungen daraus. Das Kernglas kann ein Mehrkomponentenglas sein, insbesondere kann das Kernglas eine Kombination mehrerer Oxide aufweisen, also ein oxidisches Glas sein. Quarzglas ist grundsätzlich auch als Kernglas geeignet, es weist aber im Gegensatz zu den Mehrkomponentengläsern einen sehr hohen Schmelzpunkt auf, was die Verarbeitung schwieriger und vor allem energieintensiver macht.

Bevorzugt umfasst die optische Faser eine Mantelschicht, welche den Faserkern ummantelt. In bevorzugten Ausführungsformen umfasst die Mantelschicht ein Mantelglas. Das Mantelglas kann ein Mehrkomponentenglas sein, insbesondere kann das Mantelglas eine Kombination mehrerer Oxide aufweisen, also ein oxidisches Glas sein. In anderen Ausführungsformen umfasst die Mantelschicht ein Mantelpolymer. Erfindungsgemäße Mantelpolymere sind beispielsweise Polyacrylat, Polymethacrylat, Polyurethan, Polyester, Polyamid, und Mischungen daraus.

In bestimmten Ausführungsformen ist die optische Faser eine Quarzfaser. In einer bestimmten Ausführungsform weist die Mantelschicht und/oder der Faserkern einen Anteil von mindestens 76 Gew.-% Quarz auf, weiter bevorzugt mindestens 81 Gew.-%, weiter bevorzugt mindestens 84 Gew.-%, weiter bevorzugt mindestens 88 Gew.-%, weiter bevorzugt mindestens 92 Gew.-%, weiter bevorzugt mindestens 95 Gew.-%, weiter bevorzugt mindestens 97 Gew.-%, weiter bevorzugt mindestens 98 Gew.-%. Ein höherer Quarzanteil führt zu einer erhöhten chemischen Beständigkeit sowie zu einer erhöhten Temperaturbeständigkeit.

In einer bestimmten Ausführungsform weist das Kernglas folgende Merkmale auf: Bevorzugt weist das Kernglas wenigstens 8 Gew.-%, weiter bevorzugt wenigstens 23 Gew.-%, mehr bevorzugt wenigstens 24 Gew.-% und besonders bevorzugt wenigstens 25 Gew.-% oder sogar wenigstens 26 Gew.-% SiO₂ auf. In einer besonderen Ausführungsform kann das Kernglas sogar mindestens 28,3 Gew.-% SiO₂ enthalten, ganz besonders bevorzugt mindestens 34 Gew.-% SiO₂. In einigen bevorzugten Ausführungsformen umfasst das Kernglas sogar wenigstens 35 Gew.-% SiO₂, weiter bevorzugt wenigstens 42 Gew.-%.

Bevorzugte Kerngläser dieser Erfindungen haben folgende Zusammensetzung in Gewichtsprozent:

| **Komponente** | **von** | **bis** |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

R₂O ist die Summe der Gehalte aller Alkalimetalloxide.

Eine oder mehrere der folgenden Komponenten kann enthalten sein: Cs₂O, Rb₂O, MgO, CaO, SrO, Gd₂O₃, Lu₂O₃, Sc₂O₃, Y₂O₃, In₂O₃, Ga₂O₃ und WO₃.

Folgende Komponenten sollten im Kernglas bevorzugt nicht enthalten sein oder lediglich in Konzentrationen, die durch unvermeidbare Verunreinigungen der Rohstoffe bedingt sind: TiO₂, CeO₂, Nb₂O₅, MoO₃, Bi₂O₃, PbO, CdO, TI₂O, As₂O₃, Sb₂O₃, SO₃, SeO₂, TeO₂, BeO, radioaktive Elemente und färbende Komponenten, soweit im Text nicht anders beschrieben. Insbesondere auf TiO₂ sollte verzichtet werden, weil diese Komponente für eine ausgeprägte Absorption im UV-Bereich führen kann. In bevorzugten Ausführungsformen wird auch auf die Komponente WO₃ verzichtet.

Die Komponenten TiO₂, CeO₂, Nb₂O₅ und/oder Bi₂O₃ können bis maximal 0,5 Gew.-%, bevorzugt bis 0,3 Gew.-% und besonders bevorzugt bis 0,2 Gew.-% im Kernglas enthalten sein. In einer bevorzugte Ausführungsform ist das Kernglas frei von diesen Komponenten.

Bevorzugt ist das Kernglas frei von optisch aktiven Komponenten, insbesondere Sm₂O₃, Nd₂O₃, Dy₂O₃, Pr₂O₃, Eu₂O₃, Yb₂O₃, Tb₂O₃, Er₂O₃, Tm₂O₃ und/oder Ho₂O₃. CeO₂ absorbiert im UV-Bereich, so dass bevorzugte Kerngläser kein CeO₂ enthalten.

Der Gehalt der Komponenten Erdalkalimetalloxide, La₂O₃, Ta₂O₅, ZrO₂ und HfO₂ beträgt in Summe vorzugsweise und insbesondere für Kerngläser mit Brechwerten von mehr als 1,65 wenigstens 40 Gew.-%, weiter bevorzugt wenigstens 42 Gew.-%, mehr bevorzugt wenigstens 50 Gew.-% und besonders bevorzugt wenigstens 55 Gew.-%. Wenn der Gehalt dieser Komponenten zu niedrig ist, kann der bevorzugte Brechungsindex normalerweise nicht erreicht werden. Formulierungsbedingt sollte diese Summe einen Wert von 72 Gew.-% nicht übersteigen.

In einer bestimmten Ausführungsform weist das Mantelglas folgende Merkmale auf:
Bevorzugt weist das Mantelglas einen Gehalt an SiO₂ von >60 Gew.-%, weiter bevorzugt >65 Gew.-% und besonders bevorzugt von mindestens 69 Gew.-% auf. Der SiO₂-Gehalt beträgt bevorzugt höchstens 75 Gew.-% und besonders bevorzugt bis zu 73 Gew.-%. Das Mantelglas ist tendenziell stärkeren Umwelteinflüssen ausgesetzt als das Kernglas. Ein hoher SiO₂-Gehalt verleiht bessere chemische Resistenz. Folglich ist der Gehalt an dieser Komponente im Mantelglas bevorzugt größer als im Kernglas.

Der thermische Ausdehnungskoeffizient (CTE) in einem Temperaturbereich von 20 bis 300°C kann für Faserkern und Mantel gleich oder unterschiedlich sein. Insbesondere ist der CTE unterschiedlich. Vorzugsweise ist der CTE des Mantels kleiner als der CTE des Faserkerns, vorzugsweise ist er mindestens um 1,0^{∗}10⁻⁶ /K kleiner oder besonders bevorzugt mindestens um 2,5^{∗}10⁻⁶ /K. Der Faserkern weist vorzugsweise einen CTE von 6,5^{∗}10⁻⁶ bis 10^{∗}10⁻⁶ /K auf, der Mantel einen CTE von 4,5^{∗}10⁻⁶ bis 6^{∗}10⁻⁶ /K.

Die folgende Tabelle zeigt einige bevorzugte Zusammensetzungen von Mantelgläsern, die zusammen mit den Kerngläsern verwendet werden können. Die Mantelgläser enthalten (in Gew.-% auf Oxidbasis):

| **Oxide** | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5 - 10 | 1 - 7 | 1 - 5 | 1 - 10 |
| B₂O₃ | 5 - 14 | 0 - 3 | 10 - 14 | > 15 |
| Li₂O | frei | 0-1 | 0 - 3 | < 0,1 |
| Na₂O | 0 - 10 | 8 - 20 | 2 - 8 | 0 - 10 |
| K₂O | 0 - 10 | 0 - 6 | 0 - 1 | 0 - 10 |
| MgO | 0-1 | 0 - 5 | frei | 0 - 5 |
| CaO | 0-2 | 1 - 9 | frei | 0 - 5 |
| SrO | 0 - 1 | frei | frei | 0 - 5 |
| BaO | 0-1 | 0 - 5 | frei | 0 - 5 |
| Halogen | frei | frei | frei | frei |

Als Kern- und/oder Mantelglas kommen auch Chalcogenidgläser in Frage, insbesondere die in DE 4011553 C1 und EP 0 217195 A1 offenbarten Gläser.

Der optische Faserartikel kann eine oder mehrere optische Fasern umfassen. Die optischen Fasern können hierbei ungeordnet in einem Verbund vorliegen oder in optische Faserbündel angeordnet sein. Der optische Faserartikel kann eine oder mehrere optischen Faserbündel umfassen. Die optischen Faserbündel können hierbei ungeordnet in einem Verbund vorliegen oder in Faserbündelgruppen angeordnet sein. Der optische Faserartikel kann eine oder mehrere von optischen Faserbündelgruppen umfassen.

In Ausführungsform umfasst der optische Faserartikel mindestens eine optische Faser, weiter bevorzugt mindestens zwei optische Fasern, weiter bevorzugt mindestens drei optische Fasern, weiter bevorzugt mindestens fünf optische Fasern, weiter bevorzugt mindestens sieben optische Fasern, weiter bevorzugt mindestens neun optische Fasern, weiter bevorzugt mindestens zehn optische Fasern, weiter bevorzugt mindestens elf optische Fasern. In besonders bevorzugten Ausführungsformen umfasst der optische Faserartikel mindestens 15 optische Fasern, weiter bevorzugt mindestens 50 optische Fasern, weiter bevorzugt mindestens 100 optische Fasern, weiter bevorzugt mindestens 300 optische Fasern, weiter bevorzugt mindestens 700 optische Fasern, weiter bevorzugt mindestens 1.600 optische Fasern, weiter bevorzugt mindestens 2.800 optische Fasern, weiter bevorzugt mindestens 4.700 optische Fasern.

Optische Faserbündel können mit Klebstoffen beispielsweise zu größeren Verbünden zusammengeklebt werden und/oder in Hülsen eingebettet werden. In bevorzugten Ausführungsformen weist der optische Faserartikel keine Klebstoffschicht auf. In einer Ausführungsform umfasst der optische Faserartikel zusätzlich zu der Funktionsschicht einen oder mehrere Klebstoffe, die an oder in der Funktionsschicht angeordnet sein können. Der optische Faserartikel kann dann zumindest teilweise das Reaktionsprodukt des funktionellen Silans mit einer reaktiven Klebstoffkomponente aufweisen. Vorzugsweise ist nicht der gesamte optische Faserartikel mit einem Klebstoff verklebt, sondern nur ein Teilbereich des Artikels weist das Reaktionsprodukt von funktionellem Silan und wenigstens einem Klebstoff auf. In einer Ausführungsform weist der optische Faserartikel nur auf einer Länge von höchstens 25 mm, insbesondere höchstens 15 mm oder höchstens 10 mm einen oder mehrere Klebstoffe an oder in der Funktionsschicht auf. Wird ein zu großer Abschnitt des optischen Faserartikels verklebt, so steigt die Gefahr des Faserbruchs. In einer Ausführungsform weist der optische Faserartikel auf einer Länge von wenigstens 1 mm, insbesondere wenigstens 3 mm einen oder mehrere Klebstoffe an oder in der Funktionsschicht auf.

Erfindungsgemäß verwendbare Klebstoffe mit denen optische Fasern miteinander oder mit anderen Materialien verklebt werden können sind beispielsweise Epoxidharz-Kleber, Acrylat-Kleber, Cyan-Kleber, Polyurethankleber, Silikonkleber, Phenolharzkleber, Polysulfidkleber und/oder Bismaleimidkleber. In Ausführungsformen weist der Faserartikel weniger als 10 Gew.-% Klebstoff auf, weiter bevorzugt weniger als 8 Gew.-%, weiter bevorzugt weniger als 6 Gew.-%, weiter bevorzugt weniger als 4 Gew.-%, weiter bevorzugt weniger als 2 Gew.-%, weiter bevorzugt weniger als 1 Gew.-%, weiter bevorzugt weniger als 0,8 Gew.-%, weiter bevorzugt weniger als 0,6 Gew.-%, weiter bevorzugt weniger als 0,4 Gew.-%, weiter bevorzugt weniger als 0,1 Gew.-%.

Bevorzugt weist der optische Faserartikel einen Anteil der Funktionsschicht an der optischen Faser von höchstens 10 Gew.-% auf, weiter bevorzugt von höchstens 8 Gew.-% auf, weiter bevorzugt von höchstens 6 Gew.-% auf, weiter bevorzugt von höchstens 4 Gew.-% auf, weiter bevorzugt von höchstens 2 Gew.-% auf, weiter bevorzugt von höchstens 1 Gew.-% auf, weiter bevorzugt von höchstens 0,5 Gew.-% auf, weiter bevorzugt von höchstens 0,2 Gew.-% auf, weiter bevorzugt von höchstens 0,1 Gew.-% auf. Bevorzugt weist der optische Faserartikel einen Anteil der Schutzschicht an der optischen Faser von mindestens 0,001 Gew.-% auf, weiter bevorzugt von mindestens 0,005 Gew.-% auf, weiter bevorzugt von mindestens 0,009 Gew.-% auf, weiter bevorzugt von mindestens 0,012 Gew.-% auf, weiter bevorzugt von mindestens 0,02 Gew.-% auf, weiter bevorzugt von mindestens 1 Gew.-% auf, weiter bevorzugt von mindestens 0,04 Gew.-% auf, weiter bevorzugt von mindestens 0,06 Gew.-% auf, weiter bevorzugt von mindestens 0,08 Gew.-% auf. In bevorzugten Ausführungsformen weist der optische Faserartikel einen Anteil der Schutzschicht an der optischen Faser von 0,001 bis 10 Gew.-%, weiter bevorzugt von 0,005 bis 8 Gew.-%, weiter bevorzugt von 0,009 bis 6 Gew.-%. Ist der Anteil der Schutzschicht zu gering, verliert die optische Faser an mechanischer und chemischer sowie thermischer Stabilität. Ist der Anteil der Schutzschicht zu hoch, wird der Zusammenhalt der optischen Fasern beispielsweise in einem optischen Faserbündel vermindert.

Bevorzugt weist die Funktionsschicht eine Dicke von mindestens 0,1 nm auf, weiter bevorzugt mindestens 0,9 nm, weiter bevorzugt mindestens 1,2 nm, weiter bevorzugt mindestens 1,5 nm, weiter bevorzugt mindestens 1,8 nm, weiter bevorzugt mindestens 2,1 nm, weiter bevorzugt mindestens 3 nm. Bevorzugt weist die Funktionsschicht eine Dicke von höchstens 500 nm auf, weiter bevorzugt von höchstens 200 nm, weiter bevorzugt von höchstens 100 nm, weiter bevorzugt von höchstens 50 nm, weiter bevorzugt von höchstens 20 nm. Ist die Dicke der Funktionsschicht zu gering, verliert die optische Faser an mechanischer und chemischer sowie thermischer Stabilität. Ist die Dicke der Funktionsschicht zu hoch, wird der Zusammenhalt der optischen Fasern beispielsweise in einem optischen Faserbündel vermindert.

Die optische Faser umfasst bevorzugt mindestens eine Funktionsschicht. In Ausführungsformen umfasst die optische Faser mindestens zwei Funktionsschichten, mindestens drei Funktionsschichten oder mindestens vier Funktionsschichten. Mehrere Funktionsschichten erhöhen die Biegebelastbarkeit der optischen Faser und erhöhen den Schutz vor Mikrorissen.

Bevorzugt umfasst das Verfahren zur Herstellung eines optischen Faserartikels die Schritte:
a. Bereitstellung mindestens einer optischen Faser;
b. Beschichten von mindestens einem Teil der optischen Faser mit einer Schlichte,
c. Trocknen der Schlichte.

Die optische Faser kann beispielsweise nach einem dem Fachmann bekannten Ziehverfahren wie beispielsweise aus einer Preform oder im Düsenverfahren bereitgestellt werden.

Das Beschichten der optischen Faser kann nach einem dem Fachmann bekannten Verfahren erfolgen, beispielsweise in einer Tauchwanne, durch Besprühen der Faser oder in einem Roll-to-roll-Verfahren.

Die getrocknete Schlichte bildet die Funktionsschicht. Das Trocknen der Schlichte beeinflusst die Eigenschaften der Funktionsschicht. Die Funktionsschicht wird bevorzugt an einer Gasmischung getrocknet. In bevorzugten Ausführungsformen ist die Gasmischung Luft.

Die Luftfeuchte der Gasmischung beeinflusst die Trocknung und die Materialeigenschaften der Funktionsschicht. Der Begriff Luftfeuchte meint die relative Luftfeuchte. Bevorzugt weist die Gasmischung eine Luftfeuchte von mindestens 10% auf, weiter bevorzugt von mindestens 15%, weiter bevorzugt von mindestens 20%, weiter bevorzugt von mindestens 25%, weiter bevorzugt von mindestens 30%, weiter bevorzugt von mindestens 35%. Bevorzugt weist die Gasmischung eine Luftfeuchte von höchstens 95% auf, weiter bevorzugt von höchstens 85%, weiter bevorzugt von höchstens 75%, weiter bevorzugt von höchstens 60%, noch weiter bevorzugt von höchstens 55%. In bevorzugten Ausführungsformen weist die Gasmischung eine Luftfeuchte von 10% bis 95% auf, von 15% bis 85% oder von 20% bis 60%. Ist die Luftfeuchtigkeit der Gasmischung zu niedrig, reagieren insbesondere das funktionelle Silan und/oder das optionale Alkylsilan und/oder das optionale PEG-Silan schlechter mit der Oberfläche der Faser. Hierdurch haftet die Funktionsschicht schlechter an der Faser. Ist die Luftfeuchtigkeit der Gasmischung zu hoch, trocknet die Schlichte zu langsam und es kommt zur Beeinträchtigung der mechanischen und chemischen Beständigkeit der optischen Faser. Weiterhin führt eine zu hohe Luftfeuchtigkeit zu einem zu hohen Restgehalt an Wasser und/oder wassermischbaren Lösungsmitteln in der Funktionsschicht.

Die Temperatur beeinflusst die Trocknung und die Materialeigenschaften der Funktionsschicht. Die Temperatur meint die Temperatur des die Faser während der Trocknung umgebenden Gasgemisches und/oder einer eventuellen Kontaktoberfläche, mit der eine Faser bei der Trocknung in Kontakt sein kann, z.B. eine beheizte oder unbeheizte Spule und/oder andere Fasern. Bevorzugt wird die Faserwährend der Trocknung einer Temperatur von höchstens 120°C ausgesetzt, weiter bevorzugt von höchstens 100°C, weiter bevorzugt von höchstens 80°C, weiter bevorzugt von höchstens 50°C, weiter bevorzugt von höchstens 40°C, noch weiter bevorzugt von höchstens 30°C. Bevorzugt wird die Faser während der Trocknung einer Temperatur von mindestens 8°C ausgesetzt, weiter bevorzugt von mindestens 15°C, weiter bevorzugt von mindestens 18°C, weiter bevorzugt von mindestens 20°C, weiter bevorzugt von mindestens 21 °C, noch weiter bevorzugt von mindestens 22°C. In bevorzugten Ausführungsformen wird die Faser während der Trocknung einer Temperatur von 8 bis 120°C ausgesetzt, weiter bevorzugt von 15 bis 100°C, weiter bevorzugt von 18 bis 30°C. Ist die Temperatur des Gasgemisches während der Trocknung zu hoch, kommt es zu unerwünschten chemischen Nebenreaktionen und unerwünschten Nebenprodukten. Weiterhin führt eine zu hohe Temperatur des Gasgemisches während der Trocknung zu einer schlechteren Haftung der Funktionsschicht an der Oberfläche der Faser. Ist die Temperatur des Gasgemisches während der Trocknung zu niedrig, trocknet die Schlichte zu langsam und es kommt zur Beeinträchtigung der mechanischen chemischen Beständigkeit der optischen Faser sowie zu einem zu hohen Restgehalt an Wasser und/oder wässrigen Lösungsmitteln in der Funktionsschicht.

Häufig werden Schlichten zur Härtung mit UV-Strahlung bestrahlt. Bevorzugt wird der optische Faserartikel jedoch keinem Härtungsprozess ausgesetzt und nicht durch UV-Strahlung getrocknet.

In einer Ausführungsform umfasst das Verfahren die Behandlung der getrockneten Schlichte (Trockenschlichte) mit einer flüssigen Komponente, um eine Feuchtschlichte als Funktionsschicht zu erhalten, wobei die flüssige Komponente einen Siedepunkt bei 1013 hPa von mehr als 100°C oder mehr als 200°C aufweist. Die flüssige Komponente kann ausgewählt sein aus der Gruppe bestehend aus Silikonöl, Polyethylenglykol, Alkohole (z.B. langkettige oder mehrwertige), Ester, Ether, Ketone, Acetate und Kombinationen davon. Die Trockenschlichte umfasst typischerweise weniger als 50 Gew.-%, insbesondere weniger als 35 Gew.-% der flüssigen Komponente. Die Feuchtschlichte umfasst vorzugsweise mindestens 35 Gew.-%, insbesondere höchstens 85 Gew.-% der flüssigen Komponente. In einer Ausführungsform beträgt der Anteil der flüssigen Komponente an der Feuchtschlichte zwischen 35 und 65 Gew.-%.

Erfindungsgemäß ist auch ein optischer Faserartikel mit einer solchen Feuchtschlichte als Funktionsschicht, insbesondere herstellbar nachdem hierin beschriebenen Verfahren. Der optische Faserartikel mit Feuchtschlichte kann die hierin als vorteilhaft beschriebenen Merkmale aufweisen, mit der Maßgabe, dass die Funktionsschicht einen Anteil von wenigstens 35 Gew.-% der für die Feuchtschlichte beschriebenen flüssigen Komponente aufweist.

Beim Verarbeiten von Glasfaserbündel unterscheidet man zwischen sogenannten Trocken- und Feuchtschlichten als Funktionsschicht. Bei Feuchtschlichten kleben die Einzelfasern im Bündel leicht zusammen. Damit kann erreicht werden, dass die Einzelfasern weniger stark auffächern und sich auch weniger stark elektrostatisch aufladen. Damit kann erreicht werden, dass beispielsweise das Einfädeln der Faserbündel in Hülsen deutlich erleichtert werden kann, was den Montageaufwand reduzieren hilft. Insbesondere können damit auch erhöhte Faser-Packungsdichten erzielt werden, was die Lichtstromübertragung verbessert. Zudem wird ebenfalls ein vereinfachtes Montage-Handling insbesondere bei vergleichsweise langen Lichtleitern erzielt.

Bei Verwendung von Trockenschlichten hingegen, kleben die Einzelfasern weniger zusammen. Dies ist von Vorteil, wenn beispielsweise die Einzelfasern vereinzelt und/oder im Bündel zusätzlich gemischt werden müssen (man spricht auch von einem sogenannten "Randomization-Process"), um eine homogenere Ausleuchtung zu erzielen oder, bei mehrarmigen Bündeln, die Fasern der Einzelarme möglichst gleichmäßig, d.h. statistisch gut verteilt in eine gemeinsame Endhülse montiert werden müssen. Hierbei wäre daher eine Feuchtschlichte eher hinderlich.

Vorteilhaft gemäß dem erfinderischen Ansatz ist es, dass zunächst eine Trockenschlichte als Funktionsschicht auf dem Faserartikel bereitgestellt werden kann. Erst durch Zugabe einer flüssigen Komponente, welche eine Flüssigkeit mit hohem Siedepunkt, bevorzugt > 100° C, mehr bevorzugt > 200° C ist, kann kundenspezifisch bzw. anwendungsspezifisch eine Feuchtschlichte aus der Trockenschlichte bereitgestellt werden, was deutlich Vorteile im Fertigungsablauf mit sich bringt. Grundsätzlich ist es denkbar, zunächst eine Trockenschlichte auf dem Faserartikel aufzubringen und bei Bedarf in einem nachgeschalteten Beschichtungsprozess die flüssige Komponente hinzuzufügen. Je nach Kundenwunsch bzw. je nach Anwendung kann damit die Schlichteeigenschaft gezielt eingestellt werden. Die flüssige Komponente kann dabei aus mindestens einer der nachfolgenden Bestandteile zusammengesetzt sein. Als Bestandteile der flüssigen Komponente eignen sich Silikonöl, Polyethylenglykol, Alkohole (z.B. langkettige oder mehrwertige), Ester, Ether, Ketone, Acetate und Kombinationen davon. Die Konzentration der flüssigen Komponente in der Funktionsschicht auf dem Faserartikel liegt typischerweise bei einer Trockenschlichte bei kleiner 50 Gew.-%, bevorzugt weniger als 35 Gew.-%. Bei einer Feuchtschlichte beträgt der Anteil mindestens 35 Gew.-%, typischerweise im Bereich von 35 bis 65 Gew.-%.

Bevorzugt wird der optische Faserartikel in einem Faserbündel als Lichtleiter und/oder Bildleiter verwendet. Beispielsweise kann der optische Faserartikel in Endoskopen, Inspektionskameras, Mikroskopen und/oder Spektroskopen verwendet werden.

Bevorzugt wird der optische Faserartikel in einem diagnostischen oder therapeutischen Verfahren verwendet. Beispielsweise kann der optische Faserartikel bei der Endoskopie und/oder bei Operationen mit flexiblen Inspektionskameras verwendet werden.

### Beispiele

### Beispiel E1

Zur Herstellung einer Schlichtelösung wurden zu einer Lösung aus Ethanol, VE-Wasser und Essigsäure die folgenden Komponenten (jeweils von Sigma Aldrich) in eine 1 L Laborglasflasche eingewogen und für mindestens eine Stunde auf einer laborüblichen Magnetrührplatte gerührt.

| **Komponente** | **Menge** |
|---|---|
| Stearinsäure | 1,8 g |
| Polyethylenglykol 40 | 2 g |
| Octyltrimethoxysilan | 1,2 g |
| N-[3-(Trimethoxysilyl)propyl]ethylenediamin | 0,55 g |

### Beispiele E2-E22

Zur Herstellung von Schlichtelösungen wurden zu einer Lösung aus Ethanol, VE-Wasser, optional Essigsäure und optional weiteren Lösungsmitteln wie beispielsweise Dipropylenglycolmonomethylether oder Tripropylenglycolmonomethylether die in Tabelle 1, Tabelle 2 und Tabelle 3 dargestellten Komponenten (jeweils von Sigma Aldrich) in eine 1 L Laborglasflasche eingewogen und für mindestens eine Stunde auf einer laborüblichen Magnetrührplatte gerührt. Tabelle 1 zeigt die die Komponenten ohne Lösungsmittel wie Ethanol, VE-Wasser oder Essigsäure in relativen Gew.-% der Beispiele E2-E18. PEG-Si = 2-[Methoxy(polyethyleneoxy)9-12propyl]trimethoxysilane, Ami-Si = N-[3-(Trimethoxysilyl)propyl]ethylenediamine, Alk-Si = Trime-thoxy(octyl)silane. Anschließend wurde die Schlichtelösung im Roll-to-roll-Verfahren auf optische Fasern aufgetragen und bei Raumtemperatur trocknen gelassen. Tabelle 1 zeigt auch die Ausdrückkraft und die Information, ob im Ausdrücktest die Verbindung Faser-Hülse (F-H) oder Faser-Faser (F-F) versagt hat. Des Weiteren ist die Bruchlänge angegeben.

**Tabelle 1**

| **Beispiel** | **E2** | **E3** | **E4** | **E5** | **E6** | **E7** | **E8** |
|---|---|---|---|---|---|---|---|
| PEG-Si | 60,64 | 60,60 | 60,61 | | | | |
| Ami-Si | 39,36 | 39,40 | 39,39 | 35,95 | 32,39 | 15,75 | 10,50 |
| Alk-Si | | | | | 17,61 | 34,25 | 22,83 |
| PEG400 | | | | 64,05 | 50,00 | | 33,33 |
| Stearinsäure | | | | | | 50,00 | 33,33 |
| Glycerin | | | | | | | |
| Diethylenglykol | | | | | | | |
| 1,5-Pentandiol | | | | | | | |
| Ausdrückkraft [N] | 1250 | 1250 | 1300 | 650 | - | - | 750 |
| Versagen | F_H | F-H | F-H | F-H | - | - | F-H |
| Bruchlänge [m] | | | | | 350 | 620 | |

**Tabelle 2**

| **Beispiel** | **E9** | **E10** | **E11** | **E12** | **E13** | **E14** | **E15** |
|---|---|---|---|---|---|---|---|
| PEG-Si | | | | | | | |
| Ami-Si | 12,65 | 14,17 | 10,56 | 4,35 | 2,74 | 7,92 | 6,52 |
| Alk-Si | 27,50 | 30,81 | 22,97 | 9,46 | 5,96 | 17,21 | 14,19 |
| PEG400 | 40,15 | 44,98 | 33,54 | 13,81 | 8,71 | 49,74 | 58,58 |
| Stearinsäure | 19,71 | 10,05 | | 13,81 | 8,71 | 25,13 | 20,71 |
| Glycerin | | | 32,93 | 58,58 | 73,88 | | |
| Diethylenglykol | | | | | | | |
| 1,5-Pentandiol | | | | | | | |
| Ausdrückkraft | 750 | 700 | - | - | - | 1400 | 1500 |
| Versagen | F-H | F-H | - | - | - | F-H | F-H |

**Tabelle 3**

| **Beispiel** | **E16** | **E17** | **E18** | **E19** | **E20** | **E22** | **E22** |
|---|---|---|---|---|---|---|---|
| PEG-Si | | | | | | | |
| Ami-Si | 5,55 | 2,37 | 10,06 | 4,35 | 2,74 | 4,35 | 2,74 |
| Alk-Si | 12,07 | 5,15 | 21,88 | 9,46 | 5,96 | 9,46 | 5,96 |
| PEG400 | 64,77 | 21,24 | 36,13 | 13,81 | 8,71 | 13,81 | 8,71 |
| Stearinsäure | 17,62 | 7,51 | 31,94 | 13,81 | 8,71 | 13,81 | 8,71 |
| Glycerin | | 63,73 | | | | | |
| Diethylenglykol | | | | 58,58 | 73,88 | | |
| 1,5-Pentandiol | | | | | | 58,58 | 73,88 |
| Ausdrückkraft | 1150 | 900 | - | - | - | - | - |
| Versagen | F-H | F-H | - | - | - | - | - |

Von einem Glasfaserbündel aus den mit der Schlichtelösung beschichteten optischen Fasern wurde ein IR-Spektrum gemessen. Die Messung wurde an einem durchgeführt. Hierfür wurde das Glasfaserbündel mehrfach auf den ATR-Kristall des Messgeräts gedrückt. Hierbei wurden kleine Mengen der Schlichte auf den Kristall übertragen und dann gemessen. Das FTIR-ATR-Spektrum der Probe E14 ist in Figur 2 und der Probe E18 in Figur 3 gezeigt.

### Beispiele E23 und E24

Es wurde, wie für die Beispiele E2 bis E22 beschrieben, eine Schlichtelösung unter Verwendung von Wasser und Ethanol, jedoch ohne Essigsäure hergestellt.

| **Beispiel** | **E23** | **E24** |
|---|---|---|
| Ami-Si | 8,93% | 5,6% |
| Alk-Si | 19,30% | 12,1% |
| PEG400 | 31,90% | 20,0% |
| Stearinsäure | 39,87% | 25,0% |
| Glycerin | | 50,0% |
| Ausdrückkraft | 1450 N | 1550 N* |
| Versagen | F-H | F-H |

Die Ausdrückkraft war bei beiden Schlichtelösungen sehr gut, wobei die E24 nur bei erhöhter Temperatur den hervorragenden Wert erreichte.

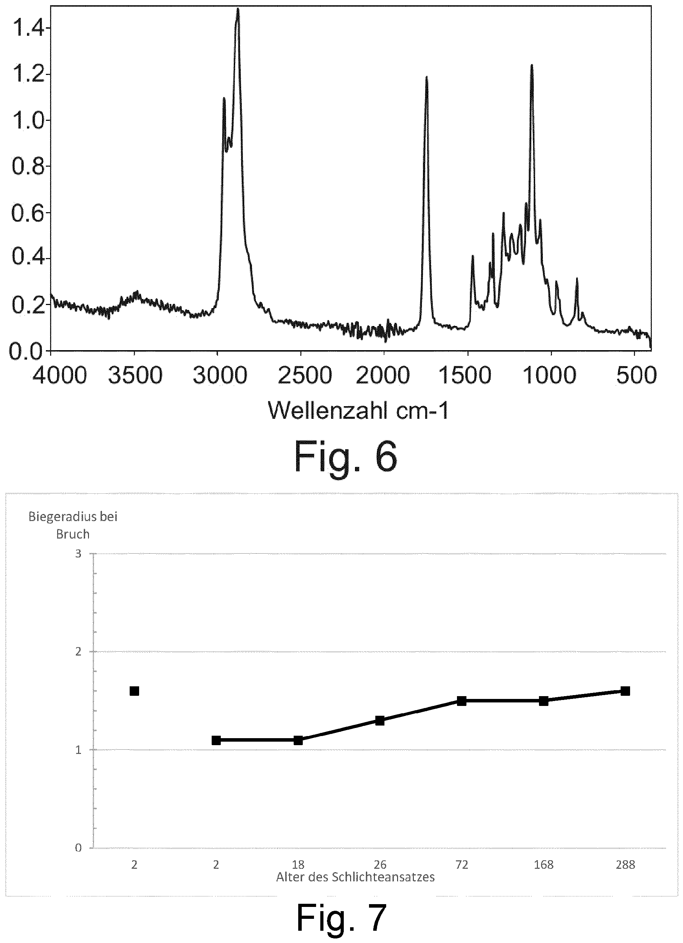

Es wurde untersucht, wie sich der Biegeradius einer mit der Schlichte E23 beschichteten Faser in Abhängigkeit der Standzeit der angerührten Schlichtelösung veränderte. Die Ergebnisse sind in Figur 7 gezeigt. Es ist erkennbar, dass der Biegeradius zunächst knapp oberhalb von 1 mm liegt und mit der Standzeit der Schlichtelösung etwas größer wird bis in den Bereich um 1,6 mm. Erst nach 288 Stunden wird ein Biegeradius erreicht, der mit dem Stand der Technik ohne funktionelles Silan (einzelner Messpunkt) vergleichbar ist.

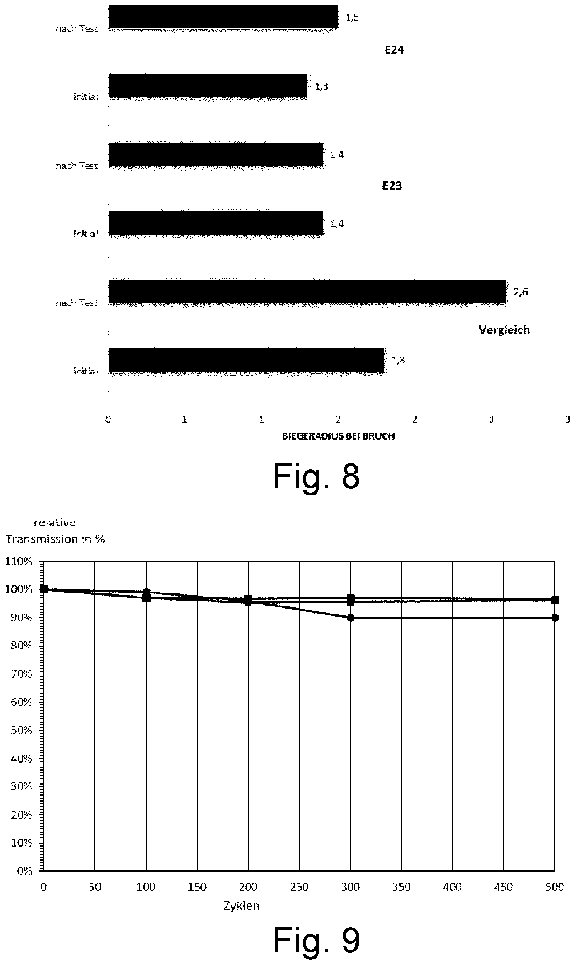

Figur 8 zeigt, wie sich die Ergebnisse des Bruchschlingentests ändern, nachdem die Fasern häufigen Temperaturwechseln unterzogen wurden. Dabei wurden die Bündel 50 mal von -40°C auf +80°C mit einer Rate von 2K/min bei 0% Luftfeuchtigkeit abgekühlt bzw. erwärmt. Es zeigte sich, dass die mit E23 und E24 behandelten Fasern nach dem Test dasselbe sehr gute Ergebnis im Bruchschlingentest zeigte, wie vor dem Test. Eine mit einer Schlichte nach dem Stand der Technik - ohne funktionelles Silan - beschichtete Vergleichsfaser zeigte deutliche Verschlechterung. Ähnlich gute Ergebnisse wurden bei Lagerung für 400 Stunden bei 85°C und 85% Luftfeuchtigkeit sowie bei 10 Tagen Lagerung in 90°C heißem Wasser erzielt.

Mit der Schlichtelösung beschichtete Faserbündel wurden darauf untersucht, in welchem Ausmaß sich ihre Transmission bei starker Reinigungsbeanspruchung verschlechterte. Dafür wurden die Faserbündel 500 Zyklen aus Reinigung und anschließender thermische Desinfektion unterzogen, wobei auf 20 Reinigungsschritten je 50 Sterilisationsschritte folgten. Figur 9 zeigt, dass sich die Transmission des mit der erfindungsgemäßen Schlichte (Quadrate = E23; Dreiecke = E24) behandelten Faser nur leicht verschlechterte auf etwa 97%, während die Vergleichsbündel (Kreise; kein funktionelles Silan) teilweise bis auf 90% abfielen. Die Daten zeigen eine exzellente chemische Beständigkeit und hydrolytische Stabilität.

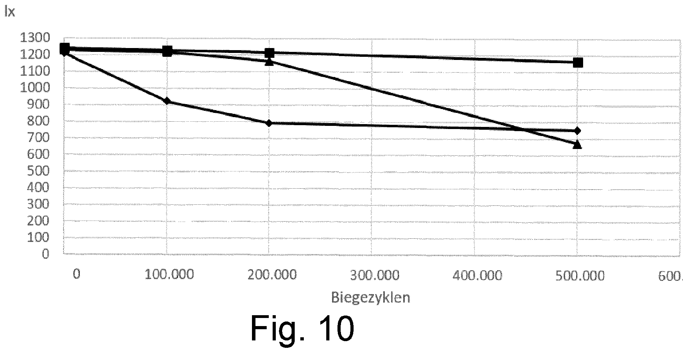

Figur 10 zeigt die Entwicklung der erzielten Beleuchtungsstärke nach wiederholtem Biegen auf einen Biegeradius von 2 mm mit Biegewinkeln von -90° bis +90°, einer Zykluszeit von 2 s und einer axialen Last von 5 N gemäß IEC 60794-1-6/E6. Es ist zu erkennen, dass die Schlichtelösung E23 (Quadrate) die besten Ergebnisse liefert. E24 (Dreiecke) ist besser als der Stand der Technik ohne funktionelles Silan (Rauten).

### Kurzbeschreibung der Figuren

**Figur 1** ist eine schematische Darstellung einer optischen Glasfaser mit einem Faserkern (1) und einem Mantel (2). Die Funktionsschicht (3) ist über kovalente Siloxanverbindungen (4) und nicht-kovalente Wasserstoffbrückenbindungen (5) mit der Oberfläche der Mantelschicht verbunden. Die Funktionsschicht umfasst beispielsweise ein Alkylsilan (6), ein Aminosilan (7), ein Polyethylenglykol (8) und eine Fettsäure (9).
**Figur 2** zeigt das FTIR-ATR-Spektrum des Beispiels E14.
**Figur 3** zeigt das FTIR-ATR-Spektrum des Beispiels E18.

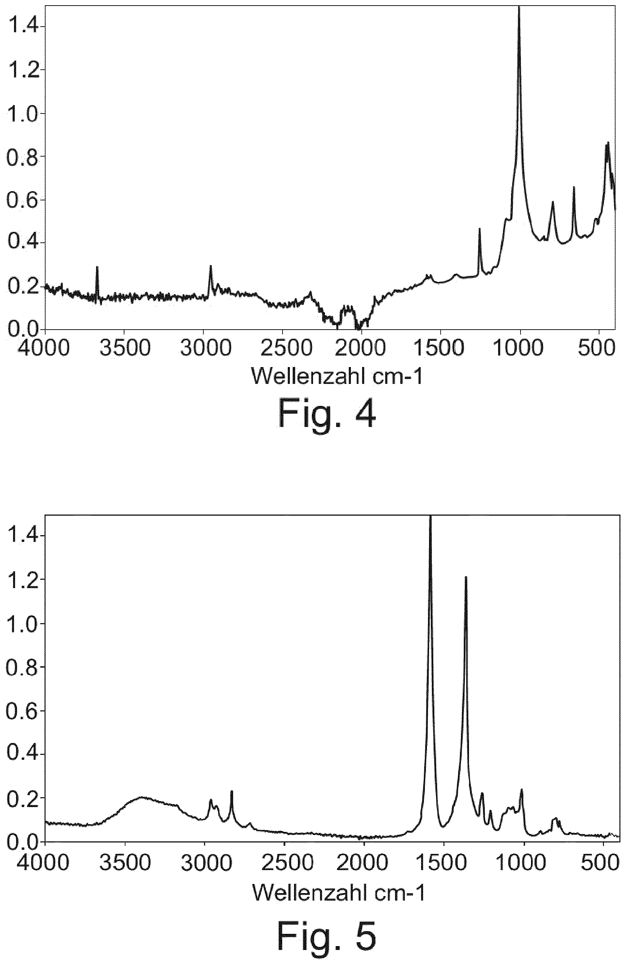

**Figur 4** zeigt das FTIR-ATR-Spektrum eines marktüblichen Faserartikels.
**Figur 5** zeigt das FTIR-ATR-Spektrum eines marktüblichen Faserartikels.
**Figur 6** zeigt das FTIR-ATR-Spektrum eines marktüblichen Faserartikels.
**Figur 7** zeigt den erreichten Biegeradius im Bruchschlingentest in Abhängigkeit von der Standzeit der Schlichtelösung im Vergleich zu einer Schlichtelösung aus dem Stand der Technik (Einzelwert).
**Figur 8** zeigt die Entwicklung des Biegeradius erfindungsgemäßer Fasern im Bruchschlingentest nach häufigen Temperaturwechseln.
**Figur 9** die Änderung der optischen Transmission erfindungsgemäßer Faserbündel nach Hunderten Reinigungs- und Sterilisationszyklen.
**Figur 10** zeigt die Entwicklung der erzielten Beleuchtungsstärke nach wiederholtem Biegen mit erfindungsgemäßer Beschichtung versehenen Fasern im Vergleich zum Stand der Technik ohne funktionelles Silan.

### Liste der Bezuqszeichen

- 1: Faserkern
- 2: Mantel
- 3: Funktionsschicht
- 4: Siloxanverbindung zwischen einem Alkylsilan der Funktionsschicht und der Oberfläche der Mantelschicht
- 5: Wasserstoffbrückenbindung zwischen einer funktionellen Gruppe der Oberfläche der Mantelschicht und einem Polyethylenglykol der Funktionsschicht
- 6: Alkylsilan
- 7: Aminosilan
- 8: Polyethylenglykol
- 9: Fettsäure

## Patentansprüche

1. Optischer Faserartikel, umfassend mindestens eine optische Faser und eine auf der Oberfläche der optischen Faser angeordnete Funktionsschicht;
wobei die Funktionsschicht mindestens ein funktionelles Silan mit folgender Strukturformel umfasst: 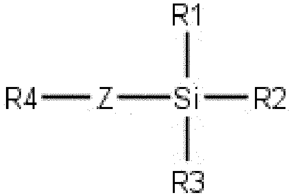
wobei Z eine verzweigte oder unverzweigte Alkylgruppe oder Arylgruppe mit 1 bis 18 Kohlenstoffatomen ist,
wobei R1, R2 und R3 unabhängig voneinander ausgewählt sind aus Wasserstoff, Sauerstoff, Alkyl, Alkyloxy, Hydroxyalkyl und Hydroxyl, und wobei eine, zwei oder drei der Gruppen R1, R2 oder R3 über eine kovalente Bindung direkt oder indirekt mit der Oberfläche der optischen Faser verbunden ist,
und wobei R4 ausgewählt ist aus, -NH₂, -NHR', -NR'R", Glycidyloxy und -SH, wobei R' und R" unabhängig voneinander ausgewählt sind aus Alkyl, Aminoalkyl, Hydroxyalkyl und -(CH₂)ₘNH₂ mit m von 1 bis 6.

2. Optischer Faserartikel nach Anspruch 1, wobei der Gehalt an fotopolymerisierten Polymeren, insbesondere an Polyacrylaten, Polymethacrylaten, Polyvinylpolymeren, Polystyrol und/oder Derivaten davon in der Funktionsschicht weniger als 1 Gew.-% bezogen auf die Masse der Funktionsschicht beträgt.

3. Optischer Faserartikel nach mindestens einem der vorhergehenden Ansprüche, wobei der Faserartikel
- weniger als 500 ppm (m/m) eines Halogenids umfasst,
- eine Ausdrückkraft von wenigstens 250 N aufweist,
- biokompatibel nach ISO10993-1:2018, USP Class VI und/oder ISO10993-5:2009 ist, und/oder
- einen Faserkern und/oder Mantel aus einem Mehrkomponentenglas aufweist.

4. Optischer Faserartikel nach mindestens einem der vorhergehenden Ansprüche, wobei die Funktionsschicht
- zusätzlich wenigstens eine Fettsäure umfasst,
- ein kovalent an die Oberfläche der Faser gebundenes Alkylsilan und/oder Polyethylenglykol-Silan umfasst, und/oder
- ein Polyalkylenoxid, insbesondere ein Polyglykol, umfasst.

5. Optischer Faserartikel nach mindestens einem der vorhergehenden Ansprüche, wobei die mindestens eine optische Faser
- im Bruchschlingentest nach DIN 58141-6:2011 einen Biegeradius von weniger als 10 mm aufweist, und/oder
- einen Faserkern und einen Mantel aufweist und die Funktionsschicht auf der Manteloberfläche angeordnet ist.

6. Optischer Faserartikel nach mindestens einem der vorhergehenden Ansprüche, wobei R4 -NHR' ist; wobei R'-(CH₂)ₘNH₂ mit m = 2 ist; und wobei Z eine unverzweigte Alkylgruppe mit 1 bis 10 Kohlenstoffatomen ist, bevorzugt von 3 bis 8 Kohlenstoffatomen ist.

7. Optischer Faserartikel umfassend mindestens eine optische Faser und eine auf der Oberfläche der optischen Faser angeordnete Funktionsschicht, wobei die Funktionsschicht im IR-Spektrum durch folgende Absorption gekennzeichnet ist:
a. ein Verhältnis der maximalen Absorptionsbandenhöhe im Bereich von 800 cm⁻¹ bis 1200 cm⁻¹ zu der maximalen Absorptionsbandenhöhe im Bereich von 1500 cm⁻¹ bis 1900 cm⁻¹ ist mindestens 2,0;
b. ein Verhältnis der maximalen Absorptionsbandenhöhe im Bereich von 2700 cm⁻¹ bis 3000 cm⁻¹ zu der maximalen Absorptionsbandenhöhe im Bereich von 1500 cm⁻¹ bis 1900 cm⁻¹ ist mindestens 2,0; und
c. ein Verhältnis der maximalen Absorptionsbandenhöhe im Bereich von 800 cm⁻¹ bis 1200 cm⁻¹ zu der maximalen Absorptionsbandenhöhe im Bereich von 2700 cm⁻¹ bis 3000 cm⁻¹ ist mindestens 1,1 und höchstens 2,0.

8. Optischer Faserartikel nach Anspruch 7, mit einem IR-Spektrum im Wesentlichen gemäß Figur 2 oder Figur 3.

9. Optischer Faserartikel nach wenigstens einem der vorhergehenden Ansprüche, mit
- einer trockenen Funktionsschicht, die einen Anteil von weniger als 35 Gew.-% einer flüssigen Komponente mit einem Siedepunkt bei 1013 hPa von mehr als 100°C aufweist, oder
- mit einer feuchten Funktionsschicht, die einen Anteil von wenigstens 35 Gew.-% einer flüssigen Komponente mit einem Siedepunkt bei 1013 hPa von mehr als 100°C aufweist.

10. Optischer Faserartikel nach wenigstens einem der vorhergehenden Ansprüche wobei das funktionelle Silan zumindest teilweise mit einem oder mehreren Klebstoffen ein Reaktionsprodukt gebildet hat.

11. Verfahren zur Herstellung eines optischen Faserartikels nach einem der vorhergehenden Ansprüche, umfassend die Schritte:
a. Bereitstellung mindestens einer optischen Faser;
b. Beschichten von mindestens einem Teil der optischen Faser mit einer Schlichte,
c. Trocknen der Schlichte.

12. Verfahren nach Anspruch 11, umfassend die Behandlung der getrockneten Schlichte mit einer flüssigen Komponente, um eine Feuchtschlichte zu erhalten, wobei die flüssige Komponente einen Siedepunkt bei 1013 hPa von mehr als 100°C aufweist.

13. Verfahren nach Anspruch 12, wobei die flüssige Komponente ausgewählt ist aus der Gruppe bestehend aus Silikonöl, Polyethylenglykol, Alkohole, Ester, Ether, Ketone, Acetate und Kombinationen davon.

14. Verwendung eines optischen Faserartikels nach einem der Ansprüche 1 bis 10 in einem Faserbündel als Lichtleiter und/oder Bildleiter, beispielsweise in einem Endoskop.

15. Optischer Faserartikel nach einem der Ansprüche 1 bis 10 zur Verwendung in einem diagnostischen oder therapeutischen Verfahren.
